# EUROPEAN PATENT APPLICATION

(11) **EP 1 707 625 A1**
(43) Date of publication of application: **04.10.2006**
(21) Application number: 04793390.8
(22) Date of filing: 29.10.2004
(51) Int. Cl.: C12N 15/00, A61K 35/28, A61K 45/00, A61P 9/00, A61P 35/00, C12N 5/00, C12Q 1/02, G01N 33/15, G01N 33/50, G01N 33/53

(54) **PROCESS FOR PRODUCING HEMATOPOIETIC STEM CELLS OR VASCULAR ENDOTHELIAL PRECURSOR CELLS**

(30) Priority: 04.12.2003 JP 2003406527
(71) Applicant: TOUDAI TLO, Ltd., Tokyo 113-0033 (JP); Medical and Biological Laboratories Co., Ltd., Nagoya-shi, Aichi 460-0002 (JP)
(72) Inventor: MIYAJIMA, Atsushi, Nerima-ku, Tokyo 1770054 (JP); TAKEUCHI, Masaki, Asaka-shi, Saitama 3510007 (JP); YAHARA, Ichiro, Yokohama-shi, Kanagawa 2220012 (JP); OKABE, Tomoya, Kamiina-gun, Nagano 3960215 (JP); ONITSUKA, Izumi, Kokubunji-shi, Tokyo 1850022 (JP)
(74) Representative: van Loon, C.J.J.
(86) International application number: PCT/JP2004/016470
(87) International publication number: WO 2005/054459

(57) **Abstract**

The present invention provides methods for producing hematopoietic stem cells or vascular endothelial precursor cells, wherein the methods comprise the step of separating PCLP1-positive cells from the hematopoietic tissues of an individual, and then culturing the obtained cells. PCLP1-positive cells obtained from the hematopoietic tissues of an individual can be cultured for a long time, and during culture they produce large quantities of hematopoietic stem cells or vascular endothelial precursor cells. The hematopoietic stem cells or vascular endothelial precursor cells obtainable by the present invention can be utilized for regenerative medicine.

## Description

### Technical Field

The present invention relates to the separation of hematopoietic stem cells or vascular endothelial precursor cells, and their uses.

### Background Art

In the development process of mammals, hematopoiesis begins as transient fetal type hematopoiesis in the yolk sac outside the embryo at around 7.5 days gestation in mice, and around three weeks gestation in humans, and mainly produces nucleated fetal type erythrocytes. Thereafter, adult type hematopoietic stem cells are produced at intraembryonic AGM region (Aorta-Gonad-Mesonephros) at around 10.5 days gestation in mice and around five weeks gestation in humans. These adult type hematopoietic stem cells migrate to the liver, where various blood cells, such as erythrocytes, lymphocytes, and platelets are produced. While the murine fetal liver matures into a digestive organ, it also functions throughout the entire fetal period as the main hematopoietic organ. In postnatal individuals, the liver loses its function as a hematopoietic tissue, matures as a digestive organ, and the bone marrow becomes the main hematopoietic tissue. In humans, hematopoiesis in the liver is observed from 12 weeks to 24 weeks gestation, and thereafter, the site of hematopoiesis shifts to the bone marrow.

Miyajima, A. *et al.* at the University of Tokyo revealed the presence of hemangioblasts, common precursors of blood cells and vascular endothelial cells, in the AGM region where adult type hematopoiesis is presumed to occur, and established methods for isolating hemangioblasts from the murine AGM region and culturing these cells. The hemangioblasts obtained using this technique could be induced to differentiate into both vascular endothelial precursor cells and blood cells by adding suitable cytokines when culturing them. Furthermore, by utilizing endothelial-like cell line (LO cells), which were derived by establishing hemangioblasts obtained from the AGM region, Miyajima *et al.* identified PCLP1 (podocalyxin-like protein 1) as a novel hemangioblast surface antigen (WO 01/34797).

PCLP1 exists in the cell membrane, and is a single-pass transmembrane glycoprotein whose extracellular region is highly glycosylated. Since the carbohydrate chain of the extracellular region of the N terminus of PCLP 1 is characteristically modified, PCLP1 is classified as a member of the sialomucin family, and the members of this family, such as CD34, CD164, CD162, CD43, and Endoglycan, are expressed in hematopoietic cells or hematopoietic microenvironments (for example, vascular endothelial cells). Molecular identification of PCLP 1 has already been carried out in the animal species below, and PCLP 1 molecules are also presumed to exist in other vertebrates.
Humans (J.Biol.Chem.272:15708-15714(1997))
Mice (Immunity.1999:11:567-578)
Rats (Accession number:AB020726)
Rabbits (J.Biol.Chem.270:29439-29446(1995))
Chickens (J.Cell.Biol.138:1395-1407(1997))

The N-terminal amino acid sequences of PCLP1 molecules are known to be poorly conserved among species (Kershaw, D. B. et al. (1997) J. Biol. Chem. 272, 15708-15714; Kershaw, D. B. et al. (1995) J. Biol. Chem. 270, 29439-29446). Homologous amino acid residues have been found at an intracellular region in the PCLP1 molecule. A PCLP1-carrying counterpart in chicken has also been reported to have hematopoietic precursor cell activity, and since its tissue localization is reported to be similar in rats, rabbits, mice, and humans, PCLP1 is considered to be a substance localized at similar sites and with similar roles between species.
[Non-Patent Document 1] J. Biol. Chem. 272: 15708-15714 (1997)
[Non-Patent Document 2] Immunity. 1999: 11: 567-578
[Non-Patent Document 3] GenBank Accession number: AB020726
[Non-Patent Document 4] J. Biol. Chem. 270: 29439-29446 (1995)
[Non-Patent Document 5] J. Cell. Biol. 138: 1395-1407 (1997)
[Non-Patent Document 6] Kershaw, D.B. et al. (1997) J. Biol. Chem. 272, 15708-15714
[Non-Patent Document 7] Kershaw, D.B. et al. (1995) J. Biol. Chem. 270, 29439-29446
[Patent Document 1] WO 01/34797

### Disclosure of the Invention

An objective of the present invention is to provide methods for separating hematopoietic stem cells or vascular endothelial precursor cells from the hematopoietic tissues of individuals.

WhenAGM region-derived cells selected using PCLP1 as the cell surface antigen are cultured, the cells are already known to differentiate into cells having the characteristics of hematopoietic stem cells or vascular endothelial precursor cells (WO 01/34797). The AGM region is a tissue formed during the developmental process of an embryo. However, there is a limit to the supply of embryos. Therefore, to utilize PCLP1-positive cells to treat humans, ideally, hematopoietic stem cells or vascular endothelial precursor cells must be isolated from more readily available cells.

Given these circumstances, the present inventors specifically used cells derived from individuals to continue their research on methods for separating hematopoietic stem cells or vascular endothelial precursor cells. As a result, the present inventors showed that hematopoietic stem cells or vascular endothelial precursor cells can also be induced from PCLP1-positive cells derived from individuals, and completed the present invention. More specifically, the present invention relates to the following techniques for producing hematopoietic stem cells or vascular endothelial precursor cells, and uses thereof.
[1] A method for producing a hematopoietic stem cell or a vascular endothelial precursor cell, wherein the method comprises the steps of:
   (1) separating a PCLP1-positive cell from a hematopoietic tissue of an individual;
   (2) inducing a hematopoietic stem cell or a vascular endothelial precursor cell by culturing the PCLP1-positive cell; and
   (3) collecting the hematopoietic stem cell or vascular endothelial precursor cell from the culture of (2).
[2] The method of [1], wherein the PCLP1-positive cell is a c-Kit-positive cell, and the method comprises the step of collecting the hematopoietic stem cell.
[3] The method of [1], wherein the PCLP1-positive cell is an erythroblast cell surface antigen-negative cell, and the method comprises the step of collecting the vascular endothelial precursor cell.
[4] The method of [3], wherein the PCLP1-positive cell is an erythroblast cell surface antigen-negative and CD45-negative cell.
[5] The method of [1], wherein the hematopoietic tissue is bone marrow.
[6] The method of [5], which comprises the step of collecting a vascular endothelial precursor cell.
[7] The method of [5], which comprises the step of collecting a hematopoietic stem cell.
[8] The method of [5], wherein the PCLP1-positive cell is a CD34-positive cell.
[9] The method of [1], wherein the hematopoietic tissue is spleen tissue.
[10] The method of [9], which comprises the step of collecting a vascular endothelial precursor cell.
[11] The method of [9], which comprises the step of collecting a hematopoietic stem cell.
[12] The method of [1], wherein step (2) is the step of co-culturing a PCLP1-positive cell with a stromal cell.
[13] The method of [12], wherein a PCLP1-positive cell and a stromal cell are co-cultured in the presence of oncostatin M (OSM), basic fibroblast growth factor (bFGF), and stem cell factor (SCF).
[14] The method of [1], wherein step (2) is the step of culturing a PCLP1-positive cell in the presence of a humoral factor present in the culture of a stromal cell.
[15] A hematopoietic stem cell or vascular endothelial precursor cell produced by the method of [1].
[16] A kit for producing a hematopoietic stem cell or a vascular endothelial precursor cell, wherein the kit comprises the following elements:
   (a) a reagent for detecting the level of PCLP1 expression; and
   (b) a medium for culturing a PCLP1-positive cell.
[17] The kit of [16], which additionally comprises (c) a stromal cell.
[18] The kit of [16], which additionally comprises (d) a reagent for detecting the level of expression of at least one cell surface antigen selected from the group consisting of an erythroblast cell surface antigen, CD45, and CD34.
[19] A method for treating a disease caused by a hematopoietic cell deficiency, wherein the method comprises the step of administering a hematopoietic stem cell obtained by the method of [1].
[20] A method for supplementing a blood cell, which comprises the step of administering a hematopoietic stem cell obtained by the method of [1].
[21] A method for treating a vascular disease, which comprises the step of administering a vascular endothelial precursor cell obtained by the method of [1].
[22] A method for detecting a regulatory effect of a test substance on angiogenic activity, wherein the method comprises the steps of:
   (1) culturing a vascular endothelial precursor cell obtained by the method of [1] with a test substance;
   (2) observing the level of growth of the vascular endothelial precursor cell; and
   (3) detecting the regulatory effect of the test substance on angiogenic activity when the level of growth is found to differ from that of a control.
[23] The method of [22], wherein an inhibitory effect on angiogenesis is detected when the level of growth is decreased.
[24] The method of [22], wherein an accelerating effect on angiogenesis is detected when the level of growth is increased.
[25] A method of screening for a substance with a regulatory effect on angiogenic activity, wherein the method comprises the steps of:
   (1) detecting the regulatory effect of a test substance on angiogenic activity as per the method of [22]; and
   (2) selecting a test substance that has a regulatory effect on angiogenic activity.
[26] An inhibitor or accelerator of angiogenesis, which comprises a substance selected by the method of [25] as an active ingredient.
[27] An anticancer agent against a cancer cell caused by angiogenesis, wherein the agent comprises, as an active ingredient, a substance with an inhibitory effect on angiogenic activity, where the substance has been selected by the method of [25].
[28] A kit for detecting a regulatory effect on angiogenic activity, wherein the kit comprises the following elements:
   a) a vascular endothelial precursor cell obtained by the method of [1]; and
   b) a medium for culturing the cell of a).

The present invention enables induction of hematopoietic stem cells or vascular endothelial precursor cells from cells derived from individuals. An important condition for widespread utilization of these cells in regenerative medicine is the ability to obtain the desired cells from materials as readily available as possible. According to the present invention, hematopoietic stem cells or vascular endothelial precursor cells can be induced from the bone marrow cells or spleen cells of individuals. Of these, bone marrow tissue can be regenerated. It is also a tissue that can be collected relatively easily. Further, bone marrow can also be collected from patients who themselves need treatment. The use of a patients' own cells is extremely effective for reducing the risk of rejection and infection by infectious pathogens.

It was also confirmed that when cultured *in vitro,* PCLP1-positive cells derived from individuals, which are cells that can be separated according to the present invention, can continuously give rise to hematopoietic stem cells or vascular endothelial precursor cells over a long time. Therefore, PCLP1 cells derived from individuals are thought to be excellent as stem cells. Furthermore, since the present invention has actualized long-term amplification of such cells, it contributes to a stable supply of hematopoietic stem cells or vascular endothelial precursor cells. Providing a stable supply of such cells is an important task that must be accomplished for transplantation therapy to be practical. Alternatively, in the development of anticancer agents that target angiogenesis, vascular endothelial precursor cells are useful as test cells for detecting regulatory effects on angiogenesis.

The present invention relates to methods for producing hematopoietic stem cells or vascular endothelial precursor cells, wherein the methods comprise the steps of:
(1) separating PCLP1-positive cells from the hematopoietic tissues of individuals;
(2) inducing hematopoietic stem cells or vascular endothelial precursor cells by culturing the PCLP1-positive cells; and
(3) collecting the hematopoietic stem cells or vascular endothelial precursor cells from the culture of (2).

In the present invention, an individual refers to an individual that has undergone tissue differentiation and can survive independently of its mother. For example, postnatal individuals are included in the individuals of the present invention. The present inventors have confirmed that PCLP1-positive cells with similar activity can be separated both from bone marrow collected from mice immediately after birth, and from bone marrow collected from mature adults. Therefore, individuals that have just been bom may also be utilized for the present invention. Needless to say, the individuals in the present invention may be adults. An adult is defined as an individual who has reached reproductive age. In the present invention, whether an individual is dead or alive does not matter, as long as amplifiable cells can be separated from that individual. Therefore, the necessary cells can be separated from an individual who is alive, brain-dead, or cardiac-dead.

PCLP1-positive cells can be separated from cells constituting a hematopoietic tissue of an individual. A discretionary tissue with hematopoietic function can be utilized as a hematopoietic tissue. Hematopoiesis refers to the production or maturation of at least one type of blood cell. Therefore, the bone marrow and spleen are included as tissues with hematopoietic function.

The present invention can be carried out on a vertebrate that carries PCLP1 as a cell surface antigen. For example, according to the present invention, the hematopoietic stem cells or vascular endothelial precursor cells of humans, mice, rats, rabbits, chickens, or such can be produced. Preferred species are humans or mice.

The hematopoietic stem cells of the present invention are cells with the pluripotency to differentiate into blood cells, and also with the ability to self-replicate. Similarly, vascular endothelial precursor cells are cells with the ability to differentiate into vascular endothelial cells. These cells can be identified by confirming a form characteristic to each of these cells, and the expression profile of each type of cell surface antigen. Alternatively, these cells can be identified by confirming whether they actually have the ability to differentiate. The specific characteristics of these cells are summarized below.

Generally, hematopoietic stem cells are cells with the pluripotency to differentiate into blood cells of all lineages, and with the ability to self-replicate. Hematopoietic stem cells of the present invention include cells that may differentiate into at least one type of blood cell. For example, cells with the pluripotency to differentiate into the cells below may be hematopoietic stem cells. Each cell can be identified by cell surface antigens, such as those indicated in parenthesis.
Myeloids (for example, Mac-1/Gr-1-positive)
Lymphoids (for example, B220/Thy-1-positive)
Erythroids (for example, erythroblast cell surface antigen-positive)
Co-culturing with stromal cells can be utilized to confirm hematopoietic stem cell activity. Various humoral factors can be added to culture systems. Examples of the humoral factors include hematopoietic system growth factors, such as stem cell factor (SCF), interleukin (IL)-3, and erythropoietin (EPO). Alternatively, phenotypes characteristic of hematopoietic stem cells can be confirmed if cells transplanted into animals with deficient hematopoiesis then reconstruct transplanted cell-derived hematopoietic stem cells or blood cells.

For rigorous verification of hematopoietic stem cells, the cells to be examined are transplanted into animals whose hematopoietic functions have been deleted by radiation, and a Long Term Repopulating-Hematopoietic Stem Cell (LTR-HSC) assay is performed, which involves long-term observation to confirm that blood cells derived from the transplanted cells are detected in all blood cell lines. When applying such an assay to the detection of human-derived hematopoietic stem cells, transgenic mice that cannot reject human cells due to severe immunodeficiency (NOD/SCID mice) can be used. The method of observing the repopulation of human blood cells in mouse bone marrow by transplanting human hematopoietic stem cells into NOD/SCID mice is called a NOD-SCID-repopulating cell (SRC) assay.

Desirable hematopoietic stem cells of the present invention have the ability to reconstruct hematopoiesis over a long time. Such hematopoietic stem cells are specifically called "long-term repopulating hematopoietic stem cells (LTR-HSC)". Herein, long-term refers to, for example, six months or more.

Furthermore, the vascular endothelial precursor cells of the present invention have been observed to be adherent cells that display a polygonal form under a phase-contrast microscope, and such cells are cells that can produce endothelial cells whose expression of low density lipoprotein (LDL) receptors can be confirmed by the incorporation of acetylated LDL. In the present invention, production of endothelial cells is preferably a growth that can be stimulated in response to OSM. More preferably, cells that are positive for cell surface antigens such as CD34, CD31, and VECadherin expressed on endothelial cells may be produced from an endothelial cell differentiation culture, in which co-culturing with OP9 stromal cells is carried out in the presence of Vascular Endothelial Growth Factor (VEGF), OSM, and such. Even more preferably, formation of a lumen may be accomplished by a three-dimensional culture using Matrigel (BD) or collagen gel. Such properties can be assayed according to known methods.

In the present invention, first, PCLP1-positive cells are separated from the cells of an individual. PCLP1-positive cells can be obtained from the hematopoietic tissues of an individual. Preferred hematopoietic tissues in the present invention are the bone marrow and spleen. For example, bone marrow is collected as follows: Bone marrow is collected as bone marrow blood from the ilium of a bone marrow donor under general anesthesia. In a conventional bone marrow transplant for adults, ordinarily 400 mL or so of bone marrow blood is collected. From the collected bone marrow blood, a mononuclear cell fraction can be separated by centrifugation under specific gravity separation, Ficoll (Pharmacia), and such. Generally, 6x 10⁸ mononuclear leukocyte cells are obtained from 400 mL of bone marrow blood.

In addition to methods for directly collecting bone marrow cells, methods for collecting these cells from the peripheral blood by driving stem cells in the bone marrow into the peripheral blood (peripheral blood stem cell transplantation) have been established. In a healthy donor, granulocyte colony stimulating factor (G-CSF) and such are used to drive hematopoietic stem cells into the peripheral blood from the bone marrow. 10 (µg/kg/day of G-CSF is administered for four to six days. Thereafter, apheresis is performed around twice such that mononuclear leukocyte cells can be recovered from the peripheral blood by a method similar to that for bone marrow. PCLP1-positive cells can also be selected from a group of cells isolated in this manner.

Methods for isolating desired cells using specific cell surface antigens as indicators are well known. More specifically, PCLP1-positive cells can be purified by reacting antibodies that recognize PCLP1 with cell populations containing PCLP1-positive cells, and then isolating antibody-bound cells using known methods. Antibodies that recognize PCLP1 are well known. Alternatively, those skilled in the art can prepare antibodies necessary for PCLP 1 detection by methods such as those indicated in the Examples. More specifically, a cDNA encoding human PCLP1 is isolated, and is expressed as a recombinant. By immunizing a suitable animal with the obtained PCLP1 recombinant, polyclonal antibodies that recognize PCLP1 can be obtained from the immunized animal. Further, monoclonal antibodies can be obtained by cloning the antibody-producing cells.

Desired cells can be separated by a cell sorter by utilizing fluorescence-labeled antibodies and using their fluorescence signal as an indicator. Multiple cell surface antigens can be used to select cells by using a combination of antibodies labeled with a pigment that fluoresces at a distinct wavelength and binds to a distinct cell surface antigen.

Cells can also be reacted with magnetic particles on which antibodies are immobilized, trapping the desired cells onto the magnetic particles. Cells bound to the magnetic particles can be separated using a magnetic instrument, such as MACS (Daiichi Pure Chemicals Co., Ltd.), thus recovering the desired cells. When selecting and separating cells using a single cell surface antigen, separation methods that use magnetic particles are convenient.

Next, the isolated PCLP1-positive cells are cultured under conditions in which hematopoietic stem cells or vascular endothelial precursor cells can be induced. The term "culture" in the present invention means *in vitro* or *ex vivo* culture. For example, hematopoietic stem cells or vascular endothelial precursor cells are known to be induced when PCLP1-positive cells isolated from the murine embryonic AGM region are co-cultured with stromal cells (WO 01/34797). Embryos are a collection of various cells in the process of differentiating into biological tissues. Therefore, cells with specific differentiation potency may be obtainable from among the cells that constitute an embryo. However, isolation of pluripotent cells from the tissues of an individual who has completed differentiation is very unlikely. Nevertheless, the present inventors confirmed that hematopoietic stem cells or vascular endothelial precursor cells can be induced by co-culture with PCLP1-positive cells separated from an individual with stromal cells. Therefore, co-culturing with stromal cells is a desirable culturing condition in the present invention. The stromal cells may be, for example, OP9 mouse stromal cells (Riken BioResource Center RCB1124). Similarly, the murine stromal cell line HESS-5 has been reported as useful for amplifying NOD/SCID mice repopulating cells (SRC) included in human umbilical-cord blood (Ando K., et al. Exp. Hematol. 28:690-699, 2000).

In addition, the murine stromal cell line M2-10B4 has also been thoroughly studied as a cell line useful for amplifying human umbilical cord blood (Cancer Res. 1996 Jun 1; 56(11): 2566-72. Engineered stromal layers and continuous flow culture enhance multidrug resistance gene transfer in hematopoietic progenitors. Bertolini F, Battaglia M, Corsini C, Lazzari L, Soligo D, Zibera C, Thalmeier K.). Preparation of stromal cells from human bone marrow, and utilization of such cells as the stromal cells of blood cells has also been reported (Int J Oncol. 2003 Oct; 23(4): 925-32. Immortalization of bone marrow-derived human mesenchymal stem cells by removable simian virus 40T antigen gene: analysis of the ability to support expansion of cord blood hematopoietic progenitor cells. Nishioka K, Fujimori Y, Hashimoto-Tamaoki T, Kai S, Qiu H, Kobayashi N, Tanaka N, Westerman KA, Leboulch P, Hara H.). Every one of these known co-culturing methods can be applied to the co-culturing methods of the present invention.

Co-culturing refers to methods of culturing PCLP1-positive cells and stromal cells in the same culture solution. The cells to be separated in the present invention are hematopoietic stem cells or vascular endothelial precursor cells. Collection of desired cells is simple if these cells are clearly different from the stromal cells in terms of adhesiveness or form (size, complexity, and such). If a clear difference is not observed between these cells, either one of the cells can be distinguished using a cell surface antigen.

Furthermore, to prevent mixing of cells, these cells can be cultured in isolation from the beginning. A known culture system that prevents contact between cells while allowing the culture solution to be shared is membrane-separated co-culturing. In membrane-separated co-culturing, a porous membrane with a pore size that allows the passage of humoral factors but blocks the migration of cells is used to culture stromal cells and PCLP1-positive cells. Humoral factors necessary for maintaining the PCLP1-positive cells and inducing hematopoietic stem cells or vascular endothelial precursor cells are provided from the stromal cells through the membrane. Since the membrane does not allow the passage of stromal cells, there is no need to worry about the stromal cells mixing with the hematopoietic stem cells or vascular endothelial precursor cells. Membrane-separated co-culturing is also a useful technique in terms of avoiding contamination by different kinds of cells.

According to the present invention, PCLP1-positive cells can be cultured in the presence of various humoral factors to aid the induction of hematopoietic stem cells or vascular endothelial precursor cells. For example, the following humoral factors are useful for inducing hematopoietic stem cells:
Oncostatin M (OSM)
Stem cell factor (SCF)
Flk2/Flt3 ligand (FL)
Thrombopoietin (TPO)
Wnt
Erythropoietin (EPO)
Interleukin-3 (IL-3)
Interleukin-6 (IL-6)
Interleukin-7 (IL-7)
Interleukin-11 (IL-11)
Soluble interleukin-6 receptor (sIL-6R)
Leukemia inhibitory factor (LIF)
Granulocyte-colony stimulating factor (G-CSF)
Stroma cell derived factor-1 (SDF-1)
Granulocyte macrophage colony stimulating factor (GM-CSF)
Macrophage colony stimulating factor (M-CSF)

The culturing methods of the present invention include methods of culturing PCLP1-positive cells in the presence of humoral factors that aid the induction of hematopoietic stem cells or vascular endothelial precursor cells, where these humoral factors are contained in the culture supernatant of stromal cells, and are useful for co-culturing PCLP1-positive cells. More specifically, the desired cells can be induced by supplying only the necessary components contained in the culture supernatant of stromal cells, and not the stromal cells themselves. The humoral factors can be supplied by adding the culture supernatant of stromal cells without further treatment. Alternatively, the proteins can be concentrated by ultrafiltration before use.

Furthermore, the culture supernatant can be fractioned, and the fractions containing the humoral factors that help induce hematopoietic stem cells or vascular endothelial precursor cells can be combined appropriately, and then used. Alternatively, the humoral factors necessary for inducing these cells can be identified. Addition of the identified humoral factor will induce a desired cell. Humoral factors are not only those derived from stromal cells, and may be genetic recombinants obtainable by expressing their genes in suitable expression systems.

The present invention is based on the novel finding that hematopoietic stem cells or vascular endothelial precursor cells can be amplified *in vitro* from PCLP1-positive cells derived from an individual. The present invention further revealed that a subfraction of PCLP1-positive cells can be separated by a combined use of another cell surface antigen in addition to PCLP1. The subfractions that can be separated by a combined use of PCLP1 with another cell surface antigen are useful for amplifying the respective cells, according to the aim. The subfractions of PCLP1-positive cells discovered by the present invention, and cells that can be amplified using that subfraction are summarized below:

| PCLP1 | CD45 | c-Kit | Erythroblast cell surface antigen | |
|---|---|---|---|---|
| + | | | - | : vascular endothelial precursor cells |
| + | - | | - | : vascular endothelial precursor cells |
| + | | + | | : hematopoietic stem cells |
| + | | - | | : erythroblast |

These cell surface antigens, which are necessary for the separation of subfractions and are used in combination with PCLP1, can be detected by antibodies that recognize each cell surface antigen, as for PCLP1. All of these cell surface antigens have already been utilized to distinguish blood cells and such. Therefore, the antibodies for detecting these cell surface antigens are commercially available. Some of the commercially available antibodies are bound to fluorescent pigments or magnetic particles. Such labeled antibodies are useful in the methods of the present invention. Of those mentioned above, mouse TER-119, human glycophorin A, human and mouse CD71, and such may be utilized as erythroblast cell surface antigens.

According to the findings of the present inventors, these subfractions are present in different proportions depending on the tissue from which the cells are derived. That is, various subfractions are included in PCLP1-positive cells separated from a particular tissue. Meanwhile, specific cells may be preferentially amplified even if a group of PCLP1-positive cells are not particularly narrowed down to subfractions. For example, PCLP 1-positive cells separated from the bone marrow of individuals can be utilized to amplify hematopoietic stem cells. Similarly, PCLP1-positive cells separated from the spleen of individuals can be utilized to amplify vascular endothelial precursor cells.

There have been attempts to amplify hematopoietic stem cells from CD34-positive cells to utilize them for regenerative medicine. Currently, CD34-positive cells are the most widely used cells for amplifying hematopoietic stem cells. For example, a method for amplifying hematopoietic stem cells by culturing CD34-positive cells separated from umbilical cord blood in the presence of a specific humoral factor has been reported (CTeda T. *et al.,* J. Clin. Invest. 105:1013-1021,2000). Analysis of the percentage of PCLP1-positive cells present in CD34-positive cells revealed the following facts.

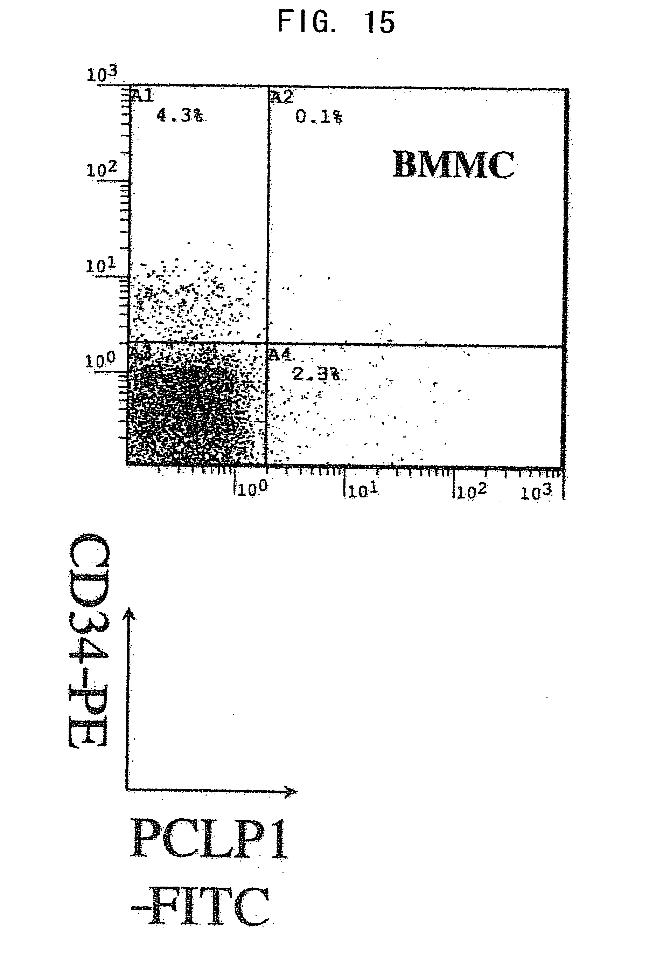

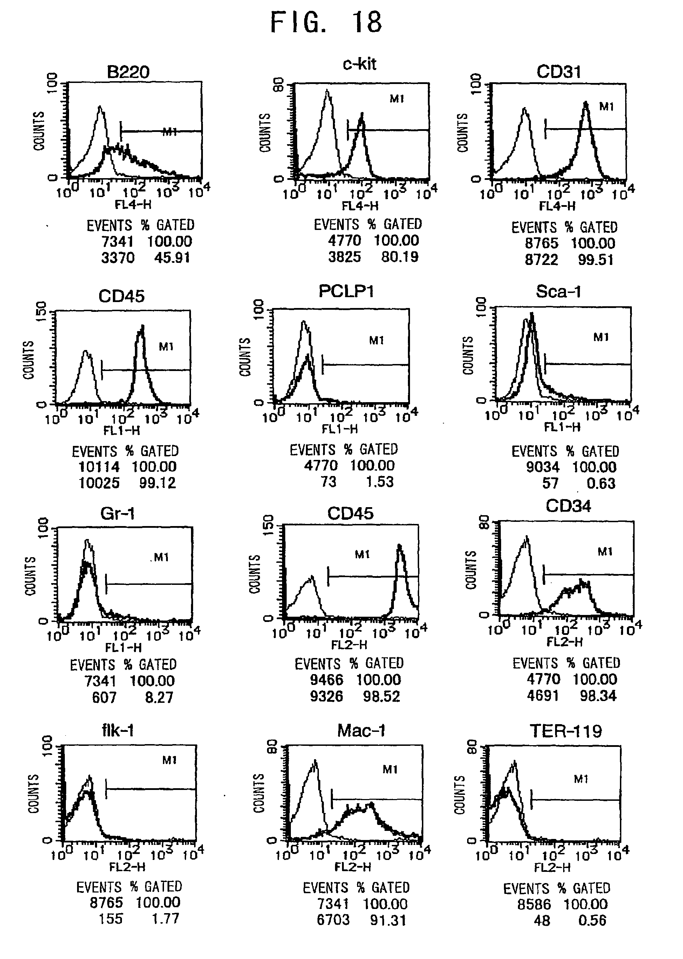

First, in the AGM region, a site where hematopoietic stem cells first emerge during the fetal period, PCLP1 was expressed by approximately 90% of CD34-positive cells (WO 01/34797). Next, the present inventors followed up on how the proportion of PCLP1-positive cells changed during development with respect to CD34-positive cells. The results confirmed that as the site of hematopoiesis gradually shifts from the AGM to the fetal liver, and then to the bone marrow, the proportion of PCLP1+ cells among CD34+ cells dramatically decreases from 50% or so in the fetal liver, to a few percent or so in the bone marrow (Fig. 18). The distribution in human bone marrow almost matched the results obtained from mice (Fig. 15).

These results show that CD34-positive cell populations in which hematopoietic stem cells are presumed to concentrate can be further fractioned by PCLP1 expression. According to the findings of the present invention, PCLP1-positive cells are thought to be more undifferentiated than PCLP1-negative cells. Therefore, cells that are PCLP1-positive and CD34-positive are preferable cell populations in the present invention. For example, cells that are PCLP1-positive and CD34-positive, and which were selected from bone marrow-derived cells, were able to maintain the function of amplifying hematopoietic stem cells for a longer time.

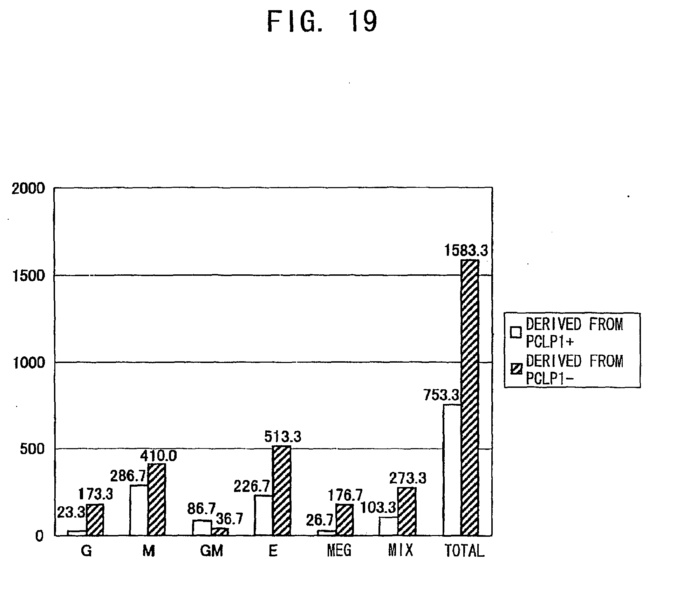

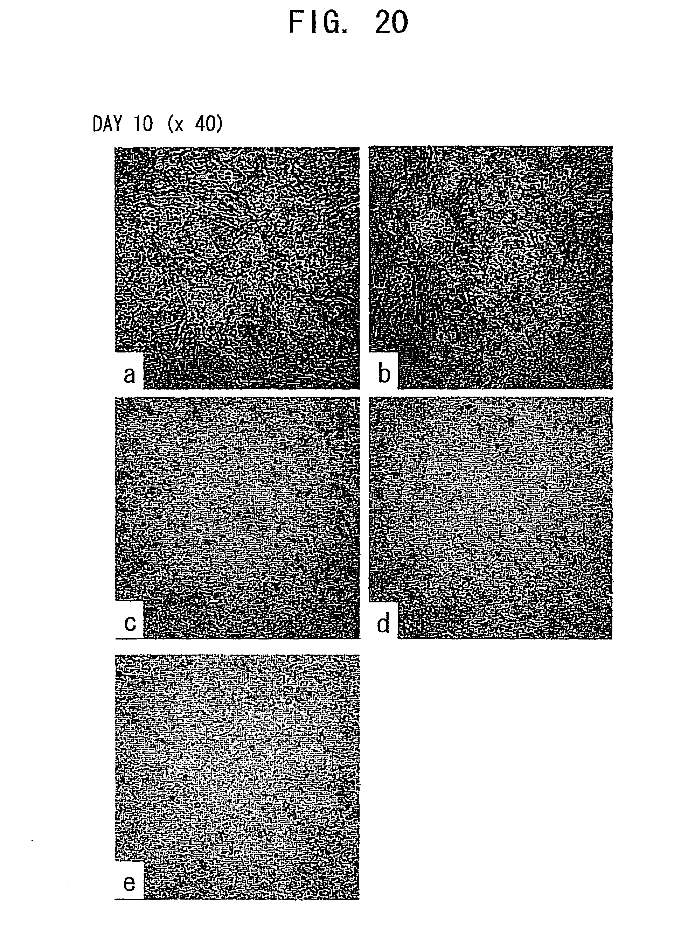

In fact, in systems for co-culturing bone marrow with stromal cells, CD34+/c-Kit+/PCLP1- cell populations start to grow blood cells at a relatively early stage, and complete the growth in a short time. On the other hand, a phenomenon has been confirmed whereby it is a long time before CD34+/c-Kit+/PCLP1+ cell populations start growing blood cells, but they continue to produce blood cells for a long time (Fig. 19). Furthermore, CD34+/c-Kit+/PCLP1- are blood cells that can be obtained in large quantities by co-culturing the latter fraction (CD34+/c-Kit+/PCLP1+) with stromal cells (Fig. 20). This shows that the PCLP1 molecule, a cell surface antigen, can be used to fractionate a less differentiated cell populations from CD34+/c-Kit+ cell populations, which are cell populations containing hematopoietic stem cells.

The present invention provides hematopoietic stem cells or vascular endothelial precursor cells amplified from PCLP1-positive cells as per the present invention. The hematopoietic stem cells or vascular endothelial precursor cells of the present invention can be utilized for various types of regenerative medicine. For example, administration of hematopoietic stem cells is effective as a method for treating blood diseases such as leukemia and aplastic anemia. In other words, hematopoietic stem cells are useful for producing therapeutic agents for blood diseases such as leukemia and aplastic anemia. Administration of vascular endothelial precursor cells is effective as a method for treating vascular diseases. In addition, vascular endothelial cells are useful for producing therapeutic agents for vascular diseases. The present invention further relates to the use of hematopoietic stem cells to develop therapeutic agents for blood diseases. Additionally, the present invention relates to the use of vascular endothelial precursor cells for developing therapeutic agents for cancers caused by vascular diseases and angiogenesis.

The hematopoietic stem cells or vascular endothelial precursor cells of the present invention can be amplified *ex vivo* or *in vitro* by culturing PCLP1-positive cells separated from the tissues of patients themselves, for example. Alternatively, desired cells can be obtained by culturing non-self tissues obtained from a donor. The desired cells, amplified through culturing, are collected, subjected to treatments such as washing, fractionation, and concentration, as necessary, and then administered to patients. The dose of each type of cell can be suitably adjusted according to the physique, sex, age, and symptoms of the patient.

Hematopoietic stem cells or vascular endothelial precursor cells obtained by the present invention can be used for therapy according to techniques similar to known allogenic bone marrow transplantation, for example. Allogenic bone marrow transplantation (BMT) is a transplantation therapy which is one of the earliest established therapeutic methods against leukemia, aplastic anemia, and congenital disorders of immunity and metabolism, and so on. During BMT, 10⁵ to 10⁶ cells/kg, for example 5 x 10⁵ cells/kg or so, of hematopoietic stem cells are ordinarily administered in a single treatment

When an allogenic transplantation of cells derived from PCLP1-positive cells provided by a donor is performed on a patient, immunosuppressive agents must be administered to prevent graft versus host disease (GVHD), antibiotics must be administered to prevent infection, and the process must be managed in a sterile room. In addition, to maintain the physical strength of the patient receiving the graft, hyperalimentation may become necessary. There is a risk of patient fever, chill, hypotension, shock, and the like during the cell transplantation; therefore, the patient's electrocardiogram is monitored, and hydrocortisone and such is pre-administered against shock. On the other hand, when the cells derived from autologous PCLP1-positive cells are transplanted, the risk of GVHD is low and administration of immunosuppressive agents is often unnecessary, however, except for this the process must be managed as for an allogenic transplantation.

For administration to patients, cells can be suspended in a discretionary medium. The hematopoietic stem cells or vascular endothelial precursor cells of the present invention may be administered after suspension in a conventionally used medium. An example of a medium suitable for dispersing the cells is physiological saline solution.

The present invention revealed that hematopoietic stem cells or vascular endothelial precursor cells can be amplified by *in vitro* culture using PCLP1-positive cells derived from individuals. Therefore, the antibodies that recognize the PCLP 1 molecule and which can be utilized to separate PCLP1-positive cells are useful as reagents for separating PCLP1-positive cells derived from individuals. Anti-PCLP antibodies can be labeled not only with a purified antibody or its variable region, but also with a discretionary substance useful for separation. More specifically, anti-PCLP1 antibodies bound to fluorescent substances, magnetic particles, enzymes, or solid phase carriers may be used as the reagents of the present invention for separating PCLP1-positive cells. More specifically, the present invention relates to the use of reagents comprising PCLP1 molecule-recognizing antibodies for separating PCLP1-positive cells.

The separated PCLP1-positive cells produce large amounts of hematopoietic stem cells or vascular endothelial precursor cells when continuously cultured under conditions of co-culture with stromal cells, or the like. Herein, various supplementary components are added to the medium, depending on the culture conditions. Medium compositions that are added with such components are useful for amplifying hematopoietic stem cells or vascular endothelial precursor cells by culturing PCLP1-positive cells. That is, the present invention provides medium compositions for amplifying hematopoietic stem cells or vascular endothelial precursor cells by culturing PCLP1-positive cells, in which the compositions comprise at least one of the combinations of components described below. Alternatively, the present invention relates to the use of a medium composition, which comprises at least one of the combinations of components described below, for amplifying hematopoietic stem cells or vascular endothelial precursor cells by culturing PCLP1-positive cells.
(1) at least one humoral factor selected from the following group:
   Oncostatin M (OSM),
   Basic fibroblast growth factor (bFGF), and
   Stem cell factor (SCF)
(2) cell surface proteins and humoral factors secreted under conditions of co-culturing PCLP1-positive cells with stromal cells

These components are added to a basal medium for culturing animal cells. Well-known basal media such as DMEM, BME, or RPMI1640 may be used for the basal medium. Alternatively, media optimized for PCLP1-positive cells by modifying such known medium compositions may also be used. The media of the present invention may also be added with animal sera. These components necessary for culturing PCLP1-positive cells can be combined in advance to form culturing kits. Co-culture media can be produced by combining stromal cells in the media. Further, culture vessels for the co-culture can also be added to the kits. An example of a culture vessel is a culture vessel for membrane-separated co-culturing.

Moreover, antibodies recognizing the PCLP1-molecule, and each element used to culture the separated PCLP1-positive cells can be combined as sets to produce PCLP1-positive cell separation systems and *ex-vivo* culturing systems. APCLP1-positive cell separation system is composed of antibodies that recognize PCLP1, and a system that utilizes such antibodies for separating PCLP1-positive cells from biological tissues. More specifically, it is composed of means to separate PCLP1-positive cells from bone marrow cells separated from an individual. PCLP1-recognizing antibodies bound to magnetic particles or solid phase carriers trap PCLP1-positive cells through contact with bone marrow cells. By collecting the antibodies which have bound to the antibodies, PCLP1-positive cells can be separated. Alternatively, by using fluorescence-labeled anti-PCLP1 antibodies, PCLP1-positive cells can be separated by a cell sorter. Furthermore, antibodies that recognize a discretionary cell surface antigen other than PCLP1 can be labeled with a fluorescent pigment different from that of the anti-PCLP1 antibodies, and multiple staining can be used to separate subfractions of PCLP1-positive cells.

More specifically, the present invention relates to kits for producing either one or both of hematopoietic stem cells and vascular endothelial precursor cells, where the kits comprise the following elements. In other words, the present invention relates to the use of kits for producing either one or both of hematopoietic stem cells and vascular endothelial precursor cells, which comprise the following elements:
(a) a reagent for detecting the expression level of PCLP1; and
(b) a medium for culturing PCLP1-positive cells

The kits of the present invention may additionally include (c) stromal cells that are useful. Alternatively, instead of stromal cells, media supplements comprising humoral factors that support the differentiation of PCLP1-positive cells into hematopoietic stem cells or vascular endothelial precursor cells may be combined. Furthermore, the kits of the present invention may additionally include (d) a reagent for detecting the expression level of an erythroblast cell surface antigen.

Vertebrates have a closed vascular system, and almost all tissues of the body are made up of close interactions between parenchymal cells characteristic of each tissue and the vascular system. Such vascular systems are formed by constructing a basic closed vascular system during the early embryonic stage (vasculogenesis), followed by construction of new blood vessel branches from the existing blood vessels (angiogenesis). Cases of abnormal angiogenesis arise in the body due to solid tumors, inflammatory diseases, diabetic retinopathy, rheumatoid arthritis, and so on. In particular, the growth of solid tumors is said to require a supply of nutrients, oxygen, and such from newly formed blood vessels. Therefore, besides methods for directly killing cancers or causing cancer regression, methods for cutting off the supply of substances required by the cancer cells are also receiving attention as strategies for developing anticancer agents. Accordingly, substances with the activity of controlling angiogenesis, and *in vitro* culture systems for selecting (screening) such substances are important for the development of pharmaceutical agents such as anticancer agents.

The vascular endothelial precursor cells obtainable by the present invention are useful for evaluating the regulatory effect on angiogenic activity. More specifically, the present invention relates to methods for detecting regulatory effects of a test substance on angiogenic activity, in which the method comprises the steps of:
(1) culturing a test substance with vascular endothelial precursor cells separated as per the present invention;
(2) observing the level of growth of the vascular endothelial precursor cells; and
(3) detecting the regulatory effect of a test substance on angiogenic activity when the level of growth is found to differ from that of a control.

In the methods of the present invention, when the level of growth of the aforementioned vascular endothelial precursor cells decreases, inhibitory effect on angiogenesis is detected. When the level of growth increases, acceleration effect on angiogenesis is detected. In the methods of this invention, the methods for culturing vascular endothelial precursor cells are not limited. For example, various media compositions for culturing animal cells are known. Such media can be used in the present invention as long as the vascular endothelial precursor cells of the present invention can be maintained. Examples of such media include Minimum Essential Medium (MEM), Basal Medium, Eagle (BME), Eagle's Minimum Essential Medium (EMEM), Dulbecco's Modified Eagle's Medium (DME), or RPMI-1640 Medium (RPMI1640). Various reinforcing components may be added to such media. More specifically, bovine serum albumin, animal serum, various humoral factors, or such can be added.

Environments for culturing vascular endothelial precursor cells include those that use a plastic culturing plate available from BD Falcon and such, a plastic plate on which substances that assist cell growth (for example, collagen or fibronectin) have been smeared, matrigel and collagengel for three-dimensional cell culture, or such, in addition to the methods of co-culturing with stromal cells, described in the present invention.

In the methods of the present invention, the level of growth of vascular endothelial precursor cells is measured. The level of cell growth can be evaluated by counting the number of viable cells. Known methods for such evaluation include methods that enable the number of viable cells to be evaluated using, as an indicator, thymidine uptake activity or the activity of reductase involved in the respiration of the cells themselves. For example, the level of cell growth can be evaluated by using an MTT Assay Kit (Roche) or such, using cell reductase activity as an indicator.

To evaluate the desired activity of a test substance on the cells obtainable by the present invention, discretionary cells can be taken as a control and used as a reference. In this case, cells cultured under conditions that do not induce the desired activity can be used as a control. More specifically, the same cells cultured in the absence of a test substance, or the same cells cultured in the presence of a component that has already been confirmed as not inducing the desired activity may be used as the control. Physiological saline solution and such may be used as the components that do not induce the desired activity.

Cells cultured in the presence of a substance that induces the desired activity may also be used as a control. When such a control is used, the magnitude of the desired activity can be compared and evaluated between the test substance and the substance used as the control.

Furthermore, based on the methods for detecting regulatory effects on angiogenic activity of the present invention, the present invention provides methods of screening for substances having such effects. More specifically, the present invention relates to methods of screening for substances that have a regulatory effect on angiogenic activity, where the methods comprise the steps of:
(1) detecting the regulatory effect of a test substance on angiogenic activity based on an aforementioned detection method; and
(2) selecting the test substance that has a regulatory effect on angiogenic activity.

Candidate substances that may be utilized in the screening methods of the present invention include, but are not limited to, purified proteins (including antibodies), gene library expression products, synthetic peptide libraries, RNA libraries, cell extract solutions, cell culture supernatants, and synthetic low-molecular-weight compound libraries.

Angiogenesis inhibitors or accelerators can be selected by the screening methods of the present invention. Inhibitors of angiogenesis are useful as therapeutic agents for diseases caused by angiogenesis. More specifically, neoplasms such as cancer, whose ability to grow is maintained by angiogenesis, can be treated by inhibiting angiogenesis. Therefore, the present invention provides anticancer agents that act against cancer cells caused by angiogenesis, where the agents comprise substances selected by the screening of the present invention as active ingredients. The present invention also relates to the use of compounds selected by the screening methods of the present invention in producing inhibitors of angiogenesis, or anticancer agents. On the other hand, substances that can be selected by the screening methods of the present invention and which have an effect in accelerating angiogenesis are useful for angiogenesis-based treatments of diseases caused by blood flow inhibition. Alternatively, the present invention relates to the use of compounds selected by the screening methods of the present invention for producing angiogenesis accelerators.

When using substances that can be isolated by the screening methods of the present invention as regulators of angiogenic activity, the substances can be used upon formulation by known pharmaceutical production methods. For example, the substances are administered to patients along with pharmaceutically acceptable carriers or vehicles (such as physiological saline, vegetable oil, suspending agents, surfactants, and stabilizers). Depending on the properties of the substances, they are administered by transdermal, intranasal, intrabronchial, intramuscular, intravenous, or oral administration. The dosage varies depending on the age, weight, and symptoms of the patient, the method of administration, and such, but those skilled in the art can suitably select the appropriate dose.

The various elements of the present invention required in the methods for detecting regulatory effects on angiogenic activity can be combined in advance and provided as kits. More specifically, the present invention relates to kits for detecting regulatory effect on angiogenic activity, where the kits comprise the elements below. Alternatively, the present invention relates to the use of the following elements in methods for detecting regulatory effect on angiogenic activity:
a) the vascular endothelial precursor cells obtained by the method described in [1]; and
b) a medium for culturing the cells of a).

Reagents for measuring the level of cell growth can be additionally combined in the kits of the present invention. Alternatively, the aforementioned PCLP1-positive cells can be separated and cultured, and these cells can be combined with the kits for amplifying the vascular endothelial precursor cells necessary for the methods of the present invention.

All prior art references cited herein are incorporated by reference into this description.

### Brief Description of the Drawings

Fig. 1 is a drawing that shows the results of analyzing PCLP1 expression in E14.5 murine fetal liver cells. The PCLP1-negative, CD45-positive fraction was defined as the leukocyte fraction (A); the PCLP1-positive, c-Kit-negative fraction was defined as the erythroblast fraction (A); the PCLP1-positive, c-Kit-positive fraction was defined as the hematopoietic stem cell fraction (B), and the PCLP1-positive, CD45-negative, and TER-119-negative fraction was defined as the endothelial precursor cell (C).
Fig. 2 is a set of photographs showing the results of co-culturing E14.5 murine fetal liver cells with OP9 stromal cells. Figs. 2a, 2c, and 2e show the culture obtained after co-culturing the PCLP1-negative and strongly CD45,TER-119-positive cells (fraction A) with OP9 cells for four, seven, and ten days, respectively. Figs. 2b, 2d, and 2f show the culture obtained after co-culturing moderately PCLP1-positive and weakly CD45, TER-119-positive or CD45,TER-119-positive cells (fraction B) with OP9 cells for four, seven, and ten days, respectively.
Fig. 2-2 is a set of photographs showing the results of co-culturing E14.5 murine fetal liver cells with OP9 stromal cells. Fig. 2-2g shows the results of subculturing moderately PCLP1-positive and weakly CD45,TER-119-positive or CD45,TER-119-positive cells with fresh OP9 cells. Figs. 2-2h, 2-2i, and 2-2j show the culture obtained after co-culturing strongly PCLP1-positive and CD45,TER-119-negative or weakly CD45,TER-119-positive cells (fraction C) with OP9 cells for three, five, and seven days, respectively.
Fig. 3 is a set of micrographs (x 100) showing the results of using a variety of endothelial cell surface antigens for immunohistological staining of endothelial-like colonies produced by co-culturing the strongly PCLP1-positive fraction with OP9 cells.
Fig. 4 is a set of graphs showing the results of collecting, on Day 10 of culture, suspended cells produced by OP9 co-culture and derived from the moderately PCLP1-positive and weakly CD45,TER-119-positive or CD45,TER-119-positive fractions, and then using flow cytometry to analyze the expression of cell surface antigens.
Fig. 5 is a set of graphs showing the results of assaying the formation of blood cell colonies when various types of cells are used. Fig. 5a shows the results of performing colony assays using a strongly PCLP1-positive fraction, moderately PCLP1-positive fraction, and weakly PCLP1-positive fraction. Fig. 5b shows the results of assaying each fraction before co-culturing with OP9 cells. Fig. 5c shows the results of performing colony assays using suspended cells produced by co-culturing each fraction with OP9 cells.
Fig. 6 is a drawing that shows the pattern of PCLP1 expression in murine fetal hematopoietic tissues.
Fig. 7 is a drawing that shows the pattern of PCLP 1 expression in hematopoietic tissues of murine individuals.
Fig. 8 is a set of photographs showing the results of co-culturing PCLP1-positive cells derived from murine individuals with OP9 cells. Figs. 8a and 8b show the endothelial cell-like colonies produced from PCLP1-positive cells derived from the spleen of an individual. Figs. 8c and 8d show the blood cells produced from PCLP1-positive cells derived from the bone marrow of an individual.
Fig. 9 is a set of graphs showing the results of performing flow cytometry to analyze the cell surface antigens of suspended cells produced by co-culturing PCLP1-positive cells derived from bone marrow of murine individuals with OP9 cells.
Fig. 10 is a set of graphs showing the results of performing colony assays using PCLP1-positive cells derived from bone marrow of murine individuals. Fig. 10a shows the results of assays using PCLP1-positive cells isolated from bone marrow. Fig. 10b shows the results of assays using suspended cells produced as a result of co-culturing PCLP1-positive cells with OP9 cells.
Fig. 11 is a drawing that shows the structure of the constructs used for expression in animal cells, in which the constructs incorporate the full-length sequence or extramembrane region sequence of human PCLP1.
Fig. 12 is a photograph of a Western blot that uses anti-myc tag to confirm the expression of full-length PCLP1 or extramembrane PCLP1 in established cell lines in which the PCLP1 protein is forcibly expressed.
Fig. 13 shows a photograph indicating the results of purifying secretory recombinant PCLP1, and performing Western blotting using anti-myc tag to confirm that the purified protein is the desired protein.
Fig. 14 is a graph showing the reactivity between transfectant CHO cells and anti-human PCLP1 monoclonal antibody.
Fig. 15 is a drawing that shows the results of separating PCLP1-expressing cells from bone marrow cells using anti-human PCLP1 monoclonal antibody.
Fig. 16 is a drawing that shows the results of further separating a CD34-positive, c-Kit-positive cell population derived from newborn mouse bone marrow into PCLP1-positive or PCLP-1 negative fractions.
Fig. 17 is a set of photographs showing the results of co-culturing newborn mouse bone marrow-derived cells with OP9 stromal cells. Figs. 17a and 17c show the culture obtained after co-culturing bone marrow-derived CD34-positive, c-Kit-positive, PCLP1-positive cells with OP9 cells for ten and 15 days, respectively. Figs. 17b and 17d show the culture obtained after co-culturing bone marrow-derived CD34-positive, c-Kit-positive, PCLP1-negative cells with OP9 cells for ten and 15 days, respectively.
Fig. 18 is a set of graphs showing the results of performing flow cytometry to analyze the cell surface antigens of suspended cells produced as a result of co-culture of OP9 cells with CD34-positive, c-Kit-positive, PCLP1-positive cells derived from newborn mouse bone marrow.
Fig. 19 is a graph showing the results of performing colony assays using suspended cells produced as a result of co-culture of OP9 cells with CD34-positive, c-Kit-positive, PCLP1-positive cells or CD34-positive, c-Kit-positive, PCLP1-negative cells derived from newborn mouse bone marrow.
Fig. 20 is a set of photographs showing the results obtained after co-culturing spleen cells of murine individuals with OP9 stromal cells for ten days. Figs. 20a and 20b each show the results obtained by co-culturing the strongly PCLP1-positive fraction. Figs. 20c, 20d, and 20e respectively show the results of culturing with the CD34-positive, c-Kit-positive, PCLP-negative fraction; the CD34-positive, c-Kit-positive, weakly PCLP1-positive fraction; and the CD34-positive, c-Kit-positive, strongly PCLP1-positive fraction.
Fig. 21 is a set of micrographs showing the condition of cells on Day 8 of co-culturing whole bone marrow cells and PCLP1-negative cells with OP9 cells (top: x 100; bottom: x 200). Cobble-stone and endothelial precursor cell-like colonies were not observed in cultures of whole bone marrow cells (left) and PCLP1-negative cells (right).
Fig. 21-2 is a set of micrographs showing the condition of cells on Day 8 of co-culturing PCLP1-positive cells with OP9 cells (top: x 100; bottom: x 200). When PCLP1-positive cells were seeded, cobble-stone and endothelial precursor cell-like colonies were observed.

### Best Mode for Carrying Out the Invention

The present invention is illustrated in detail below with reference to Examples.

### [Example 1] Isolation culture of hematopoietic precursor cells and endothelial precursor cells using fetal mouse liver

### Materials:

14.5 days pregnant C57BL/6 mice
Phosphate buffered saline (PBS)
Liver perfusion medium (GIBCO BRL)
Collagenase/Dyspase solution (GIBCO BRL)
50 µg/mL gentamicin / 15% fetal bovine serum (FBS) / DMEM (GIBCO BRL)
2% FBS/PBS
OP9 cell line (Riken BioResource Center RCB1124)
Anti-mouse CD16/32 monoclonal antibody (Pharmingen)
Biotinylated anti-mouse PCLP1 monoclonal antibody (MEL)
PE-labeled anti-mouse CD45 monoclonal antibody (Pharmingen)
PE-labeled anti-mouse TER-119 monoclonal antibody (Pharmingen)
7-AAD (Pharmingen)
Oncostatin M (OSM)
Basic fibroblast growth factor (bFGF)
Stem cell factor (SCF)
Various antibodies against mouse cell surface antigens
2% paraformaldehyde/PBS
Goat serum (Wako Pure Chemical Industries Ltd.)
Block Ace (Snow Brand Milk Products Co., Ltd.)
MethoCult (StemCell Technologies)

### Method:

### 1. Preparation of fetal liver cells

Pregnant mice were euthanized by cervical dislocation, and the uterus was removed. The uterine wall was then removed in PBS, and the fetuses were extirpated. After changing to fresh PBS, the fetal livers were extirpated under a stereoscopic microscope, and the medium was exchanged to 12 mL of liver perfusion medium per litter of fetuses (six to 12 fetuses). All of the following procedures were performed under sterile conditions.

The fetal livers were cut into small pieces using a pair of surgical scissors, and the medium was exchanged to 12 mL of Collagenase/Dyspase solution per litter of fetuses (six to 12 fetuses). This was incubated in a CO₂ incubator at 37°C for ten minutes, and then subjected to enzyme treatment. The tissue structure was destroyed by thorough pipetting using a 10-mL glass pipette to suspend the cells. This was transferred to a centrifuge tube, an equivalent amount of 50 µg/mL gentamicin/15% FBS/DMEM was added and mixed, and this was centrifuged at 4°C and 800 rpm for ten minutes. The supernatant was removed, and 15 mL of an ice-cooled hemolysis buffer (0.1 M NH₄Cl/16.5 mM Tris) was added per litter of fetuses (six to 12 fetuses), the cells were loosened by gently pipetting two to three times, and this was left on ice for nine minutes for hemolysis. An equivalent amount of 50 µg/mL gentamicin/15% FBS/DMEM was added, and this was centrifuged at 4°C and 800 rpm for ten minutes. The collected cells were diluted in 10 mL of 50 µg/mL gentamicin/15% FBS/DMEM, and this was passed through a 70 µm cell strainer. Dead cells were stained with trypan blue, and the number of stained cells was counted using a hemocytometer.

### 2. Antibody reaction

Anti-mouse CD16/32 monoclonal antibody was diluted 100 times with 50 µg/mL gentamicin/15% FBS/DMEM. 1 mL of this solution was added per 1 x 10⁷ cells, this was mixed, then left on ice for 15 minutes, and non-specific binding of antibodies was inhibited by FcR blocking. About 1 x 10⁶ cells were placed into each of three tubes, and the antibodies below were added to the respective tubes and then mixed to produce isotype controls and samples for fluorescence correction. Each of the antibodies was added such that they were diluted 100 times.
Tube 1: biotinylated rat IgG2a and PE-labeled rat IgG2a
Tube 2: biotinylated anti-mouse CD45 monoclonal antibody and PE-labeled rat IgG2a
Tube 3: biotinylated rat IgG2a and PE-labeled anti-mouse CD45 monoclonal antibody

Biotinylated anti-mouse PCLP1 monoclonal antibody, PE-labeled anti-mouse CD45 monoclonal antibody, and PE-labeled anti-mouse TER-119 monoclonal antibody were added to the remaining cells such that they were each diluted 100 times, and this was mixed to prepare the samples. After adding the antibodies, the respective cells were left on ice for 30 minutes.

The isotype controls, fluorescence correction samples, and samples were each washed with ice-cooled 2% FBS/PBS. The isotype controls, fluorescence correction samples, and samples were then each rediluted in streptavidin-APC diluted 50 times with 2% FBS/PBS, and then left on ice for 30 minutes. They were then washed with ice-cooled 2% FBS/PBS. Cells were diluted at 1 x 10⁶ cells per 5 µL of 7-AAD, and then left at room temperature for five minutes. The cells were diluted in 2% FBS/PBS or diluted in PBS to 5 x 10⁶ to 1 x 10⁷ cells/mL, and then transferred to a cell separator tube.

### 3. Sorting (Cell separation)

Using isotype controls and fluorescence correction samples, the sensitivity of each parameter of the cell separator was adjusted and fluorescence corrections were made. With reference to the fluorescence intensity of the isotype control, each of the cell populations below were gated, and the cells were fractionated into tubes containing 50 µg/mL gentamicin/15% FBS/DMEM mixed with 10 µg/mL OSM, 1 µg/mL bFGF, and 100 µg/mL SCF.
Strongly PCLP1-positive and CD45, TER-119-negative or weakly
CD45,TER-119-positive
Moderately PCLP1 positive and weakly CD45, TER-119-positive or
CD45,TER-119-positive
PCLP1-negative and strongly CD45, TER-119-positive

The fractionated cells were reanalyzed to confirm that they were purely fractionated according to the gates that were set The number of obtained cells was counted using a hemocytometer.

### 4. Co-culturing of separated cells with stromal cells

On a 10-cm dish or 6-well plate, OP9 stromal cells were cultured in 50 µg/mL gentamicin/15% FBS/DMEM until about 70% to 90% confluent. Immediately before plating the sorted cells onto this dish or plate, the medium was exchanged for a medium containing cytokines (10 µg/mL OSM, 1 µg/mL bFGF, and 100 µg/mL SCF). The strongly PCLP1-positive and CD45, TER-119-negative or weakly CD45, TER-119-positive fraction were plated onto a 6-well plate at about several hundred to 5000 cells per well, and the moderately PCLP1-positive and weakly CD45, TER-119-positive or CD45, TER-119-positive fraction and the PCLP1-negative and strongly CD45, TER-119-positive fraction were each plated at 20 000 cells per well. The cells were cultured in a CO₂ incubator at 37°C under 5% CO₂ partial pressure. Blood cell production in each of the fractions was observed under a microscope for several weeks, starting the day after plating.

### 5. Colony Assay

The sorted cells were added to MethoCult to a concentration of 1000 cells/mL, to which a final concentration of 50 µg/mL gentamicin was added and mixed. 1 mL of this solution was placed into each well of a 6-well plate using a 1-mL syringe and an 18G needle. To retain moisture, 1 mL of sterilized distilled water or PBS was placed onto a corner of the plate, and the cells were cultured in a CO₂ incubator at 37°C under 5% CO₂ partial pressure. On Day 9 of culturing, the colonies were observed under a microscope, the colony types were classified, and then counted.

### 6. Analysis of blood cells

After several days of OP9 co-culture, and after producing a sufficient amount of suspended cells, the suspended cells were exclusively collected into a centrifuge tube, taking care to avoid OP9 contamination. The obtained cells were used to analyze cell surface antigen expression using flow cytometry, and to measure growth activity of the blood cells using colony assays.

### 7. Analysis of endothelial-like colonies

The dish was washed with PBS, taking care not to detach the cells. The cells were immobilized with 2% paraformaldehyde/PBS and then 5% goat serum/BlockAce (Snow Brand Milk Products Co., Ltd.) was used for blocking (for one hour at room temperature). A primary antibody reaction was carried out overnight at 4°C, and then washing was carried out using PBS. A fluorescence-labeled anti-mouse IgG antibody (secondary antibody) reaction was carried out for one hour at room temperature, and after washing with PBS, the cells were observed under a microscope.

### Results:

### 1. Analysis of cell populations in fetal liver

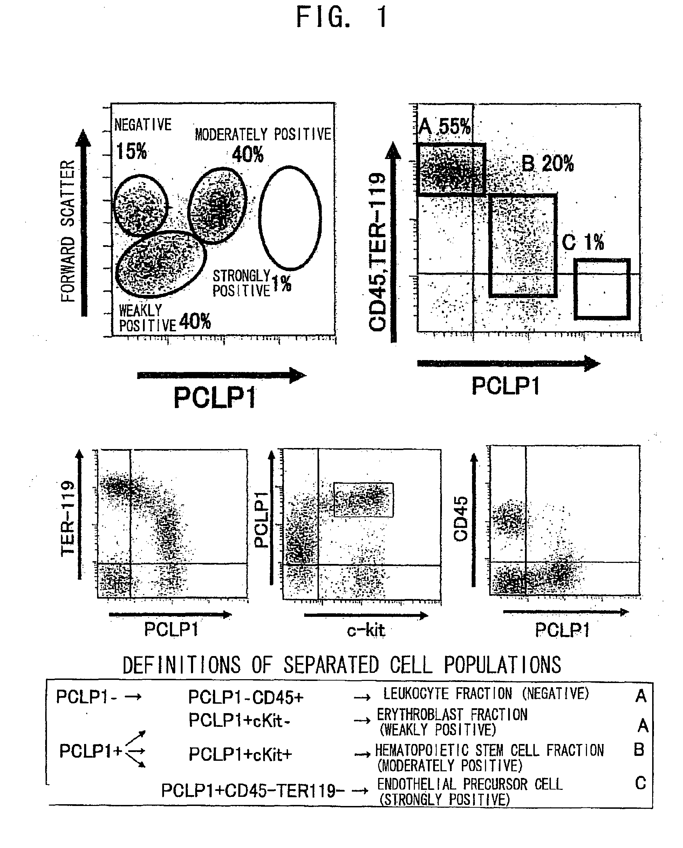

The results showed that the expression af PCLP1 in E14.5 fetuses can be classified into four groups according to the intensity of expression: highly positive (approximately 1%), moderately positive (approximately 40%), weakly positive (approximately 40%), and negative (approximately 15%) (Fig. 1). The weakly PCLP1-positive cell population and PCLP1-negative cell population (fraction A) were strongly CD45, TER-119-positive; the moderately PCLP1-positive cell population (fraction B) was weakly CD45, TER-119-positive or CD45, TER-119-positive; and the strongly PCLP1-positive cell population (fraction C) was CD45, TER-119-negative or weakly CD45, TER-119-positive (Fig. 1).

### 2. Results of culturing separated cells

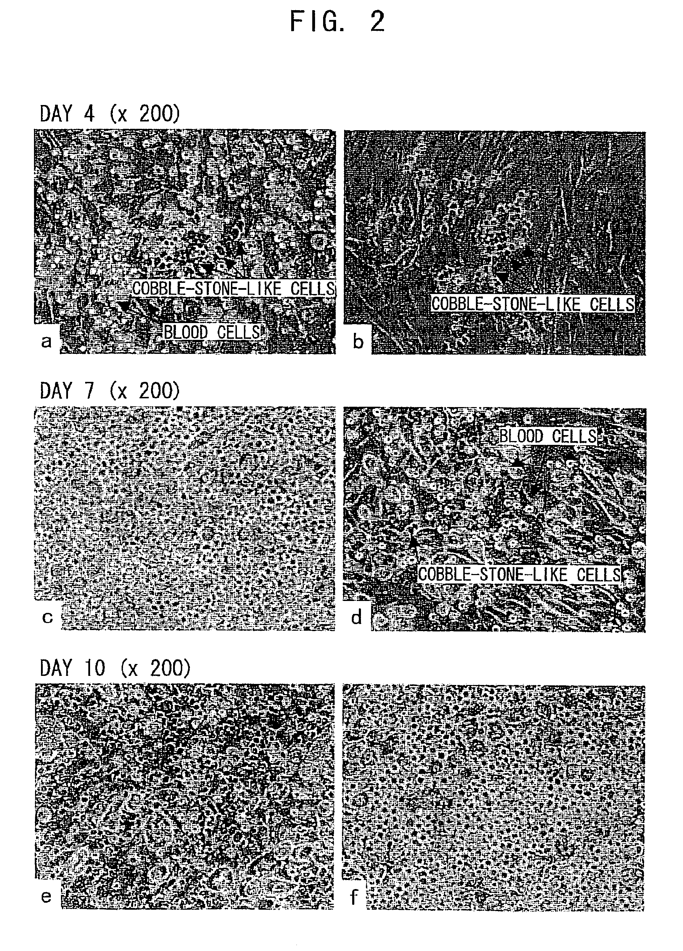

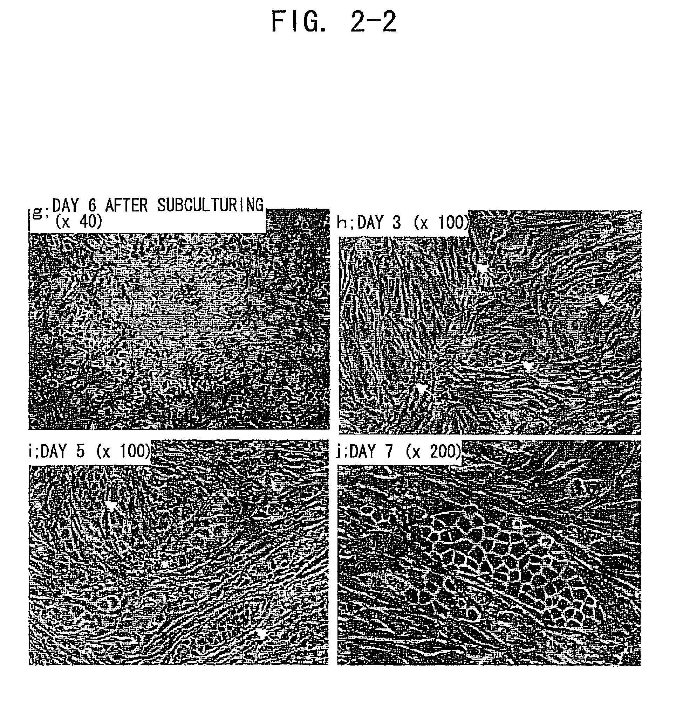

Observation of each OP9 co-cultured fraction under a phase-contrast microscope showed that on Day 2 to 3 of culture many cobble-stone area-forming cells (CAFCs), which appear black because they are under OP9 stromal cells, were observed in the wells plated with PCLP1-negative and strongly CD45,TER-119-positive cells, and formation of many white glowing suspended cells around these CAFCs was observed (Fig. 2a). As for the wells plated with PCLP1-negative cells, CAFCs were observed in the wells plated with moderately PCLP1-positive and weakly CD45, TER-119-positive or CD45, TER-119-positive cells, but the white glowing suspended cells were not produced (Fig. 2b).

In the wells plated with moderately PCLP1-positive and weakly CD45, TER-119-positive or CD45, TER-119-positive cells, the white glowing suspended cells started to grow around the CAFCs from approximately Day 7 to 10 of culture (Fig. 2d). Blood cell growth observed when culturing PCLP1-negative and strongly CD45, TER-119-positive cells gradually slowed at about ten days to a few weeks. However, blood cell growth observed when culturing moderately PCLP1-positive and weakly CD45, TER-119-positive or CD45, TER-119-positive cells continued for a few weeks or more, and these cells could be further cultured by subculturing using fresh OP9 cells (Fig. 2-2g).

On the other hand, at about Day 3 to 6 of culturing in wells plated with strongly PCLP1-positive and CD45, TER-119-negative or weakly CD45, TER-119-positive cells, endothelial-like colonies formed and grew at a rate of approximately 10% of the number of plated cells (Figs. 2-2h-j).

### 3. Analysis of adherent cells formed by culturing

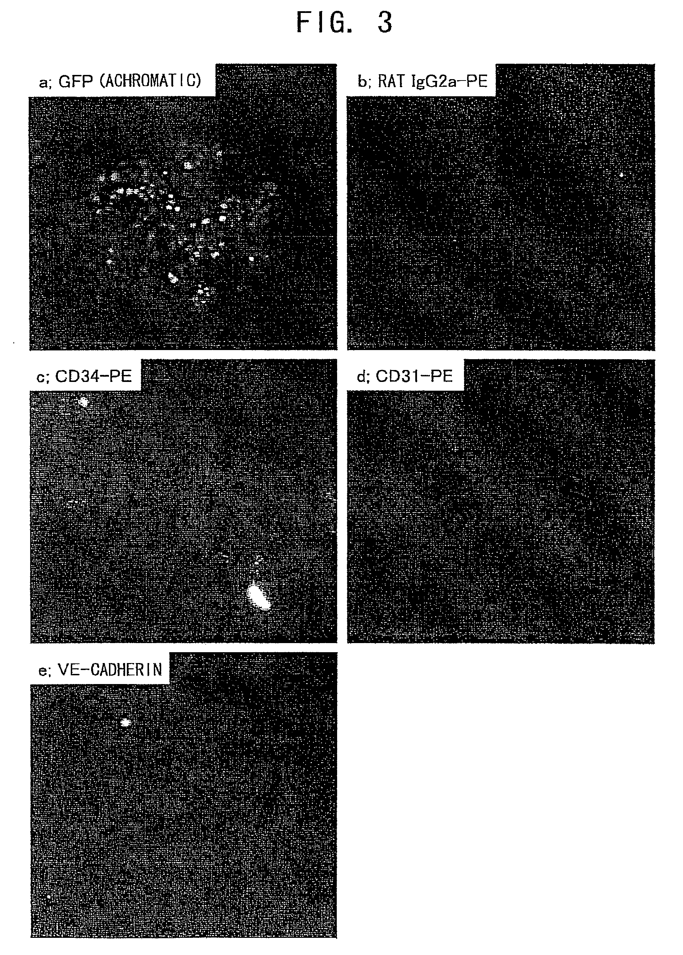

The endothelial-like colonies produced by co-culturing the strongly PCLP1-positive fraction with OP9 were immunohistologically stained using various types of endothelial cell surface antigens. As a result, CD34, CD31, and VE-Cadherin were clearly stained as compared to the isotype control (Fig. 3).

### 4. Results of blood cell analysis

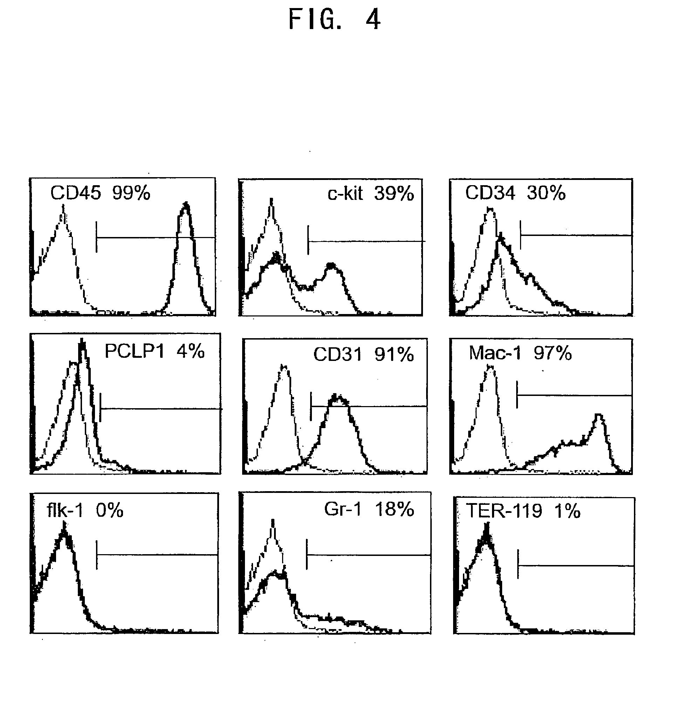

Suspended cells, derived from the moderately PCLP1-positive and weakly CD45, TER-119-positive or CD45, TER-119-positive fractions produced by OP9 co-culture, were collected on Day 10 of culture, and when the expression of cell surface antigens was analyzed by flow cytometry, nearly 100% of the cells expressed leukocyte cell surface antigen CD45, and cell surface antigens of hematopoietic stem cells and hematopoietic precursor cells, such as CD34, c-Kit, Sca-1, and CD31, were highly expressed (Fig. 4).

### 5. Colony assay results

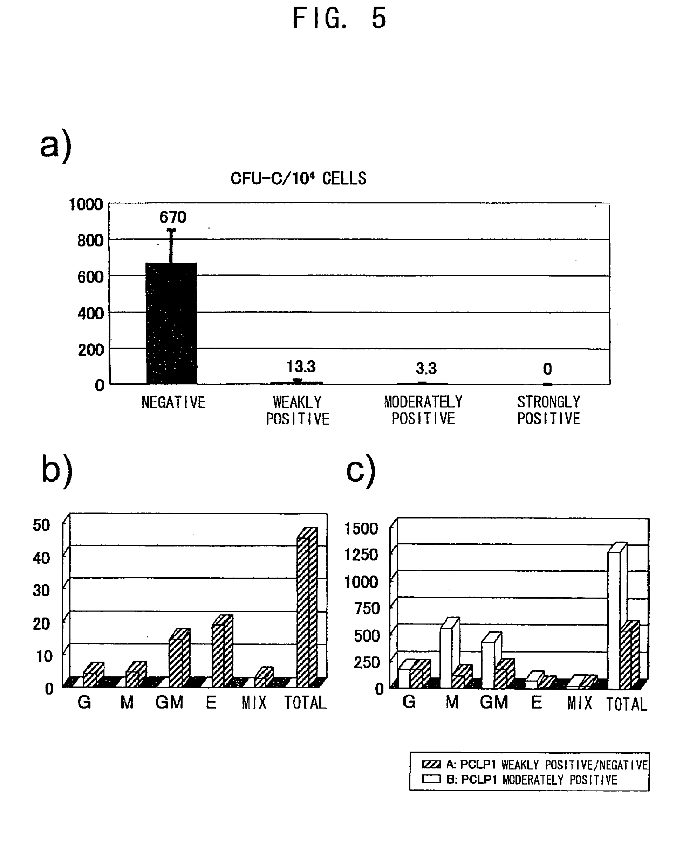

In the colony assays, the Colony Forming Units (CFU) were taken as the number of colonies formed for every 10 000 cells plated, and CFU-C indicates the total number of colonies formed. CFU- followed by a letter of the alphabet indicates the number of colonies formed by each type of differentiated blood cell, and G, M, Meg, E, and Mix respectively refer to granulocytes, monocytes and macrophages, megakaryocytes, erythroblasts, and a mixture of all types of cells. Blood cell colony formation was hardly observed from the strongly PCLP1-positive, moderately PCLP1-positive, and weakly PCLP1-positive fractions. The number of colonies formed from the PCLP1-negative fraction was CFU-C=670 per 10 000 cells, and that in the entire liver was CFU-C=63.3 per 10 000 cells (Fig. 5a). Furthermore, the number of colonies formed from the PCLP1-negative and strongly CD45, TER-119-positive fraction (fraction A) was CFU-G=4.3, CFU-M=4.7, CFU-GM=14.7, CFU-Meg=0.7, CFU-EM=18.3, CFU-Mix=3.0, and CFU-C=45.7 per 10 000 cells (Fig. 5b).

### 6. Results of colony assays on blood cells produced by culturing

Colony assays using suspended cells produced by OP9 co-culture showed that the number of colonies formed from suspended cells derived from the moderately PCLP1 positive and weakly CD45, TER-119-positive or CD45, TER-119-positive fraction and the PCLP1-negative and strongly CD45-, TER-119-positive fraction were CFU-C=1276.7 and CFU-C =543.3, respectively, and the colony forming ability of both types of cells increased remarkably compared to that observed prior to OP9 co-culture (Fig. 5c).

### 7. Results of subculturing the adherent cells produced by culturing

Immunostaining of the endothelial-like colonies produced by OP9 co-culture revealed the expression of CD34 and VE-Cadherin, which are endothelial cell surface antigens. The endothelial-like colonies together with OP9 were trypsinized, then the cells were dispersed and replated on to fresh OP9, once again causing formation and growth of endothelial-like colonies.

### Discussion:

The fraction of PCLP1-negative and strongly CD45, TER-119-positive cells is a cell population comprising blood cell precursor cells that can immediately provide functional blood cells; therefore, this fraction was thought to begin active blood cell growth from the early stages of culture. In contrast, the fractions of moderately PCLP1-positive and weakly CD45, TER-119-positive or moderately CD45, TER-119-positive cells comprise juvenile blood cells that are still in the process of differentiation, and thus it is some time before blood cell growth starts when these cells are co-cultured with OP9 stromal cells. Accordingly, these fractions are thought to begin blood cell precursor cell production later compared to the PCLP1-negative and strongly CD45, TER-119-positive cell fraction, and this blood cell growth continued for a long time.

Since suspended cells derived from moderately PCLP1-positive cells were the only cells that could be subcultured, and they could sustain longer-term production of blood cells, this fraction is likely to contain blood cell stem cells that can self-replicate. Further, since both the PCLP1-negative and strongly CD45, TER-119-positive cell fraction and the moderately PCLP1 positive and weakly CD45, TER-119-positive or CD45, TER-119-positive cell fractions showed remarkably different colony forming abilities before and after OP9 co-culture, and since both showed a considerable increase in colony forming ability after OP9 co-culture, OP9 co-culturing appears to strongly induce blood cell differentiation and growth.

The results of cell surface antigen expression analysis by flow cytometry were that the strongly PCLP1-positive cells were negative or weakly positive for known endothelial cell surface antigens CD34, CD31, and Flk-1. However, when these fractions were OP9 co-cultured, they frequently formed endothelial-like colonies that were positive for the endothelial cell surface antigens CD34 and VE-Cadherin. Therefore, it was not until after co-culturing with stromal cells that the strongly PCLP1-positive cell fraction differentiated into endothelial cells, and these fractions may be cell populations comprising endothelial precursor cells that may acquire the properties of endothelial cells. This indicates that using anti-PCLP1-antibodies enables the separation of more juvenile endothelial precursor cells not obtainable using existing cell surface antigens.

The above showed that by combining the level of PCLP1 expression with information on CD45 and TER-119 expression, a cell fraction comprising blood cell precursor cells, a cell fraction comprising more juvenile blood cell stem cells, and a cell fraction comprising endothelial precursor cells can each be separated. It also showed that co-culturing with OP9 stromal cells can strongly induce *in vitro* blood cell differentiation and growth, and that this growth activity can be maintained for a long time.

### [Example 2] Isolation culture of hematopoietic precursor cells and endothelial precursor cells using tissues of murine individuals

### Materials:

Newborn C57BL/6 mice
PBS
Collagenase/Dyspase solution (GIBCO BRL)
50 µg/mL gentamicin/15% FBS/DMEM (GIBCO BRL)
2% FBF/PBS, OP9 cell line
Anti-mouse CD16/32 monoclonal antibody (Pharmingen)
Biotinylated anti-mouse PCLP1 monoclonal antibody (MBL)
APC-labeled anti-mouse c-Kit monoclonal antibody (Pharmingen)
FITC-labeled anti-mouse CD34 monoclonal antibody (Pharmingen)
Streptavidin-APC (Molecular Probes)
7-AAD (Pharmingen), Oncostatin M (OSM)
Basic fibroblast growth factor (bFGF)
Stem cell factor (SCF)
Various antibodies against mouse cell surface antigens
MethoCult (Stem Cell Technologies)

### Methods:

### 1. Preparation of tissue cells (spleen, bone marrow) of individuals

The spleen and bone marrow were extirpated from newborn mice. The spleen or bone marrow from a litter of fetuses (six to 12 fetuses) was soaked in 12 mL of Collagenase/Dyspase solution, cut into small pieces using a pair of surgical scissors, incubated at 37°C for ten minutes in a CO₂ incubator, and then subjected to enzyme treatment. After thorough pipetting using a 10-mL glass pipette, the cells were dispersed, transferred to a centrifuge tube, an equivalent amount of 50 µg/mL gentamicin/15% FBS/DMEM was added and mixed, and this was centrifuged at 4°C and 800 rpm for ten minutes. The supernatant was removed, the cells were resuspended in 50 µg/mL gentamicin/15% FBS/DMEM, and the number of cells was counted using a hemocytometer.

### 2. Antibody reaction

Anti-mouse CD16/32 monoclonal antibody was diluted 100 times with 50 µg/mL gentamicin/15% FBS/DMEM, and 0.1 mL of this solution was added per 1 x 10⁶ of spleen or bone marrow cells. This was then mixed, the mixture was left on ice for 15 minutes, and non-specific antibody binding was inhibited by FcR blocking. About 1 x 10⁵ cells were placed into each of four tubes, and the antibodies below were added to the respective tubes. This was then mixed to produce isotype controls and samples for fluorescence correction. Each of the antibodies was added such that they were diluted 100 times.
Tube 1: FITC-labeled rat IgG2a, PE-labeled rat IgG2a, and biotinylated rat IgG2a
Tube 2: FITC-labeled anti-mouse CD45 monoclonal antibody, PE-labeled rat IgG2a, and biotinylated rat IgG2a
Tube 3: FITC-labeled rat IgG2a, PE-labeled anti-mouse CD45 monoclonal antibody, and biotinylated rat IgG2a
Tube 4: FITC-labeled anti-rat IgG2a, PE-labeled rat IgG2a, and biotinylated anti-mouse CD45 monoclonal antibody

Biotinylated anti-mouse PCLP1 monoclonal antibody, APC-labeled anti-mouse c-Kit monoclonal antibody, and FITC-labeled anti-mouse CD34 monoclonal antibody were added to the remaining cells such that they were then each diluted 100 times, and this was mixed to prepare the samples. After adding the antibodies, the respective cells were left on ice for 30 minutes. The isotype controls, fluorescence correction samples, and the samples were each washed with ice-cooled 2% FBS/PBS. The isotype controls, fluorescence correction samples, and samples were each rediluted in streptavidin-APC diluted 50 times with 2% FBS/PBS, and then left in ice for 30 minutes. They were then washed with ice-cooled 2% FBS/PBS. Cells were diluted at 1 x 10⁶ cells per 5 µL 7-AAD, and then left at room temperature for five minutes. The cells were diluted in 2% FBS/PBS or diluted in PBS to a concentration of 2 x 10⁶ to 5 x 10⁶ cells/mL, and then transferred to cell separator tubes.

### 3. Sorting (analysis and separation of cells)

Using isotype controls and fluorescence correction samples, the sensitivity of each parameter of the cell separator (cell sorter) was adjusted and fluorescence corrections were made. The samples were developed according to the fluorescence intensity of PCLP 1 and cell size (FS peak). With reference to the fluorescence intensity of the isotype control, the strongly PCLP1-positive, moderately PCLP1-positive, weakly PCLP1-positive, and PCLP1-negative regions in the sample were each gated, and in the combined staining for CD34 and c-Kit, the relationship between each cell surface antigen was analyzed and gates were set for these surface antigens. The groups of sorted cells were fractionated in tubes containing 50 µg/mL gentamicin/15% FBS/DMEM, and the number of cells were counted using a hemocytometer.

### 4. In vitro culturing of separated cells

On a 10-cm dish or 6-well plate, OP9 stromal cells were cultured in 50 µg/mL gentamicin/15% FBS/DMEM until about 70% to 90% confluent, and then a suitable number of sorted cells were plated to this culture after replacing the medium with a medium containing cytokines (10 µg/ml OSM, 1 µg/ml bFGF, 100 µg/ml SCF). The co-culture dish was incubated in a CO₂ incubator at 37°C under 5% CO₂ partial pressure. Blood cell production in each of the fractions was observed under a microscope for several weeks, starting the day after plating.

### Results:

### 1. Analysis of expression pattern in the spleen

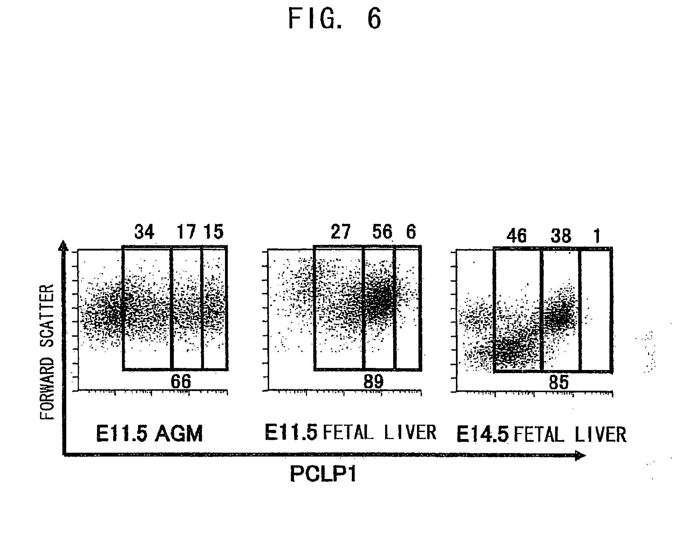

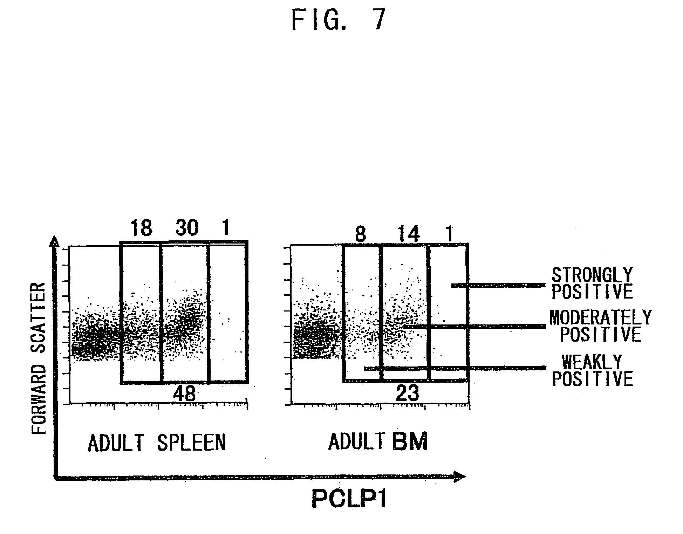

The results showed that expression of PCLP 1 in the spleen can be categorized into four groups, according to expression intensity: strongly positive (PCLP1++; approximately 1%), moderately positive (PCLP1+; approximately 30%), weakly positive (PCLP1low; approximately 18%), and negative (PCLP1-; approximately 51%) (Fig. 7). This pattern of PCLP1 expression was similar to the pattern of PCLP1 expression in E14.5 fetal liver (Figs. 6 and 7). Cell fractions that were CD34-positive and c-Kit-positive were detected as a distinct group constituting 5% or so, and expression of PCLP1 in this fraction was mostly negative, although a slight distribution was observed over the weakly positive to positive regions.

### 2. Co-culturing of spleen cells with stromal cells

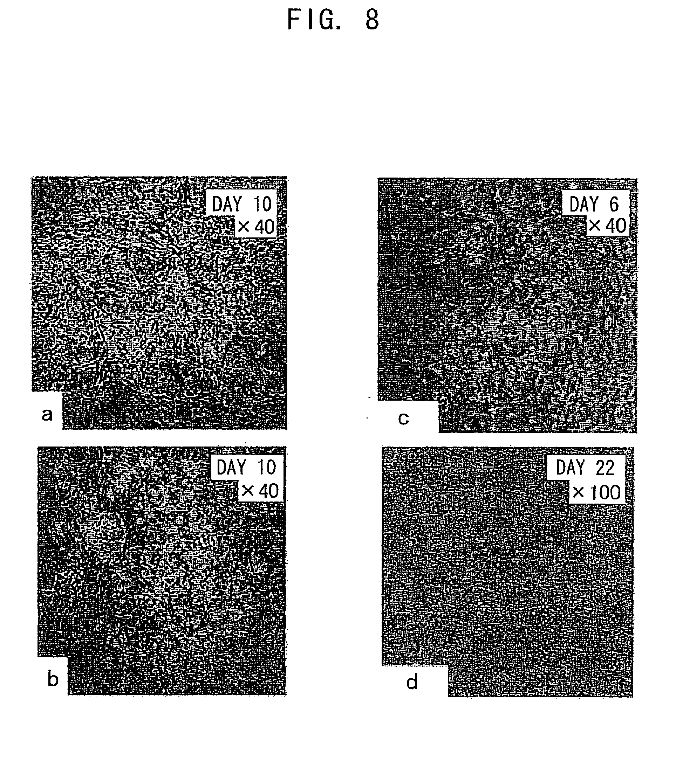

By Day 10 of culture the strongly PCLP1-positive fraction was found to be forming many endothelial cell-like colonies, morphologically similar to the endothelial cell-like colonies formed from an OP9 co-culture of strongly PCLP1-positive fetal liver cells (Figs. 8a, b).

### 3. Analysis of expression pattern in bone marrow

The results showed that the expression of PCLP1 in bone marrow could be categorized into four groups, according to expression intensity: strongly positive (approximately 1 %), moderately positive (approximately 14%), weakly positive (approximately 8%), and negative (approximately 77%) (Fig. 7). In mice of sufficient age, the proportion of moderately positive cells tended to decrease to about 1%, but in terms of their ability to produce blood cells, a trend similar to that of juvenile mice was observed.

### 4. Co-culturing of bone marrow cells with stromal cells

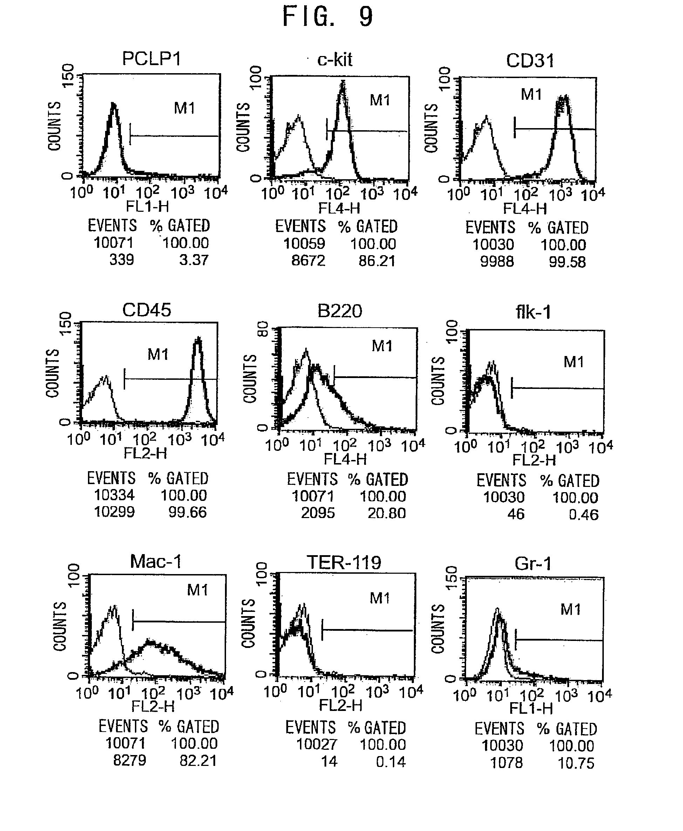

When PCLP1-positive cells were co-cultured with OP9 stromal cells, the suspended blood cells were observed to form in clusters within one week of culturing, and cobble stone-like cells could also be recognized. On Day 11 of culturing, the suspended cells grew vigorously, appearing as a sea of clouds, and OP9 cells could not be observed at all (Fig. 8c). Thereafter, blood cells were continuously produced for over a month (Fig. 8d). On Day 13 of OP9 co-culture, the suspended cells produced from the OP9 co-culture were collected, and flow cytometry was used to analyze the expression of cell surface antigens. As a result, nearly 100% of the cells expressed CD45, and hematopoietic stem cell and the hematopoietic precursor cell surface antigens c-Kit and CD31 were expressed with high frequency (Fig. 9).

### 5. Colony assays

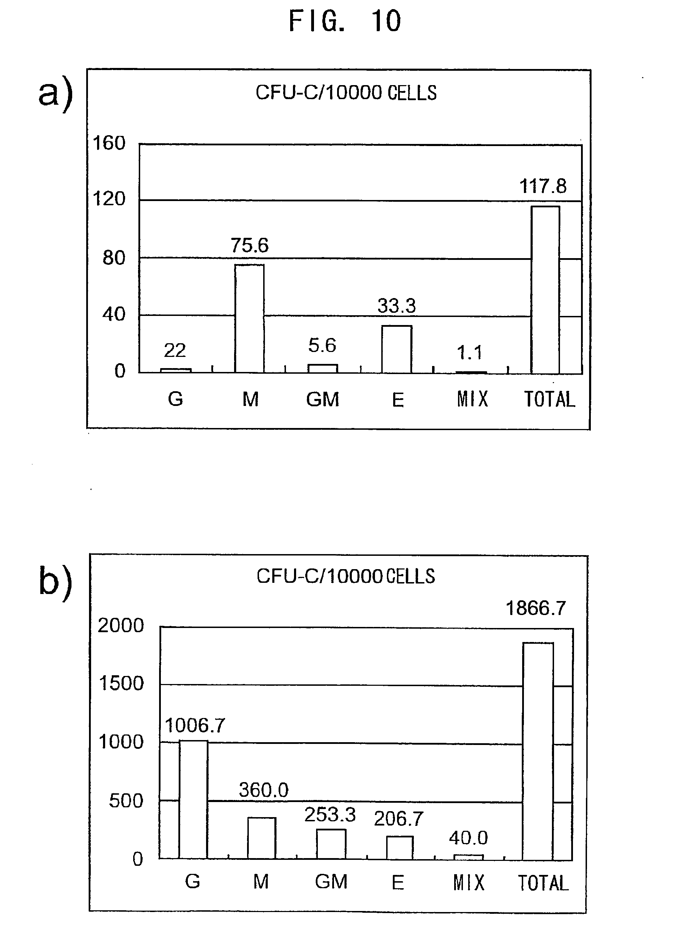

PCLP1-positive cells were separated from the bone marrow and then subjected to colony assays, showing activity of CFU-G=2.2, CFU-M=75.6, CFU-GM=5.6, CFU-E=33.3, CFU-Mix=1.1, and CFU-C=117.8 per 10 000 cells (Fig. 10). On Day 13 of co-culturing PCLP1-positive cells with OP9 cells, the suspended cells produced by OP9 co-culture were collected and then subjected to colony assays. The results were CFU-G=1006.7, CFU-M=360.0, CFU-GM=253.3, CFU-E=206.7, CFU-Mix=40.0, and CFU-C=1866.7 per 10 000 cells (Fig. 10).

### Discussion:

The results showed that strongly PCLP1-positive cells exist in the tissues of individuals, although in low frequency, and OP9 co-culturing produces endothelial-like colonies that are morphologically similar to those produced from the strongly PCLP1-positive cells of fetal liver. Furthermore, as for fetal liver, blood cell growth in PCLP1-positive cells was activated later than in PCLP1-negative cells. The above results showed that during the developmental process of a fetus, as the site of hematopoiesis shifts from the AGM region, where adult-type hematopoiesis begins, to the liver and tissues of an individual, a strongly PCLP1-positive cell fraction and a moderately PCLP1-positive cell fraction are consistently present in each of the hematopoietic organs, and throughout the transition, the strongly PCLP1-positive cell fraction consistently includes a high frequency of endothelial precursor cells, and the moderately positive cells include hematopoietic stem cell-like juvenile cells that continuously produce blood cells for a long time.

The results also demonstrated that the use of a stromal cell co-culturing system, such as the OP9 co-culturing system, enables *ex vivo* or *in vitro* growth of immature precursor cells derived from the tissues of individuals over a long time. CD34-positive and c-Kit-positive cell fractions are cell fraction with hematopoietic stem cells and hematopoietic precursor cell fractions concentrated to a certain degree, but the PCLP1-positive cell population within this fraction is very probably a subfraction which is at a different stage of blood cell differentiation. That is, within the hematopoietic stem cell and hematopoietic precursor cell fractions, the cell populations expressing PCLP1 may be more juvenile. However, the colony forming ability of suspended cells after co-culturing with stromal cells tended to be higher for cells derived from PCLP1-negative fractions. Since only the PCLP1-positive fractions continued to produce blood cells for several weeks thereafter, at this point the PCLP1-positive fractions may not have reached the stage of blood cell differentiation with the highest growth activity.

### [Example 3] Separation of PCLP1-positive cells from human bone marrow and confirmation of reactivity

### Methods:

### 1. Cells

Human bone marrow monocytes (BMMC) were purchased as frozen cells from Cambrex (Japanese supplier: Sanko Junyaku Co., Ltd.) and then used. The CHO cells used for gene transfer were purchased from the Riken BioResource Center and then subcultured in F12HAM medium (SIGMA) containing 10% FBS (MBL) and 50 µg/mL gentamicin (GIBCO).

### 2. Gene transfection and establishment of a cell line in which the human PCLP1 molecule is forcibly expressed

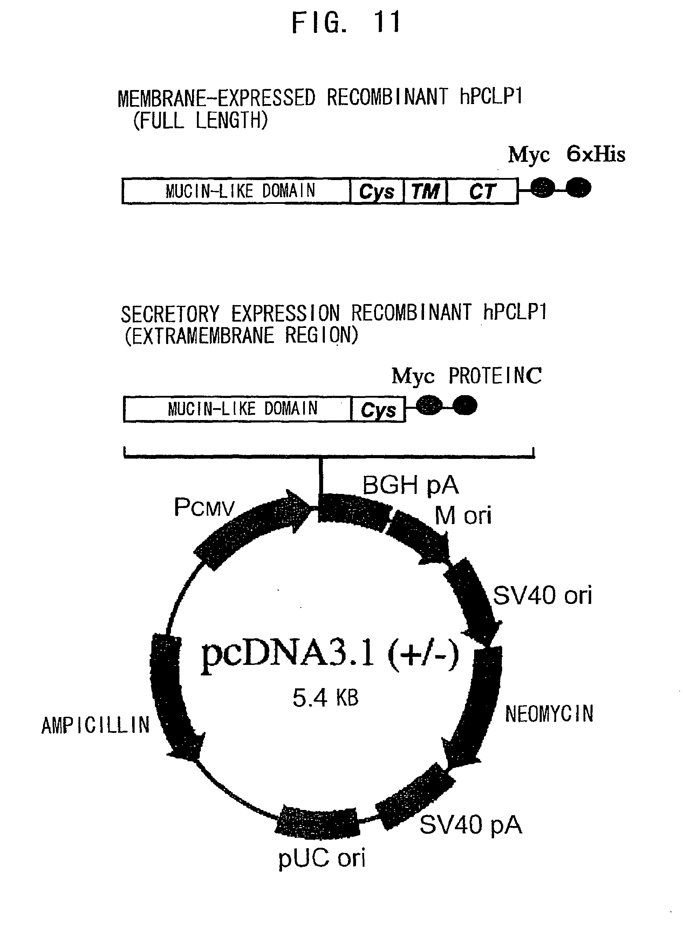

Human PCLP 1 cDNA was cloned from a human placenta library, and the full length sequence and extramembrane region sequence were used to make constructs for expression in animal cells using the pcDNA3.1 vector (Invitrogen). The structure of the constructs is shown in Fig. 11. The membrane-expressed recombinant derived from the full length PCLP 1 gene can be expressed on the surface of cells such as 293T and CHO, and can be used to evaluate antibody reactivity. The secretory expression recombinant derived from the extramembrane region of the PCLP1 gene is expressed and secreted as a recombinant protein into the culture medium of insect cells or animal cells, and this protein can be used as an immunogen and for ELISA. CHO cells that reached 70% confluency were transfected using TransIT Kit (PanVera) with 6 µg of each of the constructs. The transfected cells were exclusively selected by culturing in a 10% FBS-F12HAM medium containing 700 µg/mL of G418 (GIBCO), and cell lines stably expressing the membrane-bound (clone: 12C) and secretory (clone: 18E) PCLP1 molecules were both obtained.

### 3. Purification of secretory recombinant PCLP1

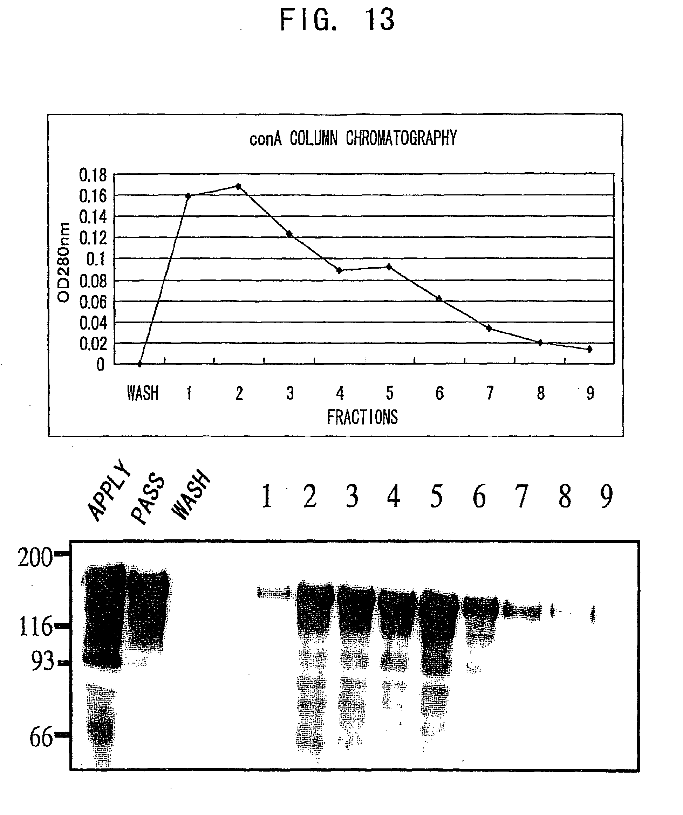

The secretory PCLP1-expressing cell line 18E was cultured for one week in 1 L of 10% FBS-F12HAM medium, the culture was dialyzed overnight at 4°C against PBS, and then purified using a WGA-Sepharose column (Amersham). Recombinant PCLP1 that adhered to the column was eluted with PBS containing 200 mM *N*-acetylglucosamine, the eluted fractions were dialyzed again against a phosphate buffer solution (pH7.4), and then adsorbed onto DEAE-Sepharose (Amersham). The recombinant adsorbed onto the column was eluted with PBS containing 1 M NaCl, then the eluted fractions were combined, diluted 5-fold using a phosphate buffer (pH7.4), and then adsorbed onto a ConA Sepharose (Amersham). The recombinant adsorbed onto the column was eluted using PBS with a concentration gradient of 0-200 mM α-dimethylglucose, and the fractions reactive to myc tag antibody (MBL) were collected as purified products (Fig. 13). PBS was used to equilibrate and wash the column.

### 4. Confirming protein expression in the transgenic cells and in the purified protein

Western blotting was carried out to confirm that the transgenic cells and the purified protein are the desired recombinant PCLP1 protein. After subjecting the sample to electrophoresis on a 10% polyacrylamide gel, the proteins were electrically transferred from the gel onto a PVDF membrane (Millipore). The transferred membrane was blocked overnight in 5% skim milk-PBS at 4°C. The membrane was washed with PBS, then reacted with a 2000-fold diluted anti-myc tag antibody (MBL) at room temperature for one hour. After washing with PBS, this was reacted with a 3000-fold diluted peroxidase-labeled anti-mouse IgG (H+L) antibody (MBL) at room temperature for one hour. The membrane was washed thoroughly with PBS, color developed with SuperSignal coloring substrate, and an X-ray film (Fuji Film) was then exposed using the resultant signals.

### 5. Production of monoclonal antibodies

100 µL of complete adjuvant (Iatron) was pre-injected to Balb/c mice, and one day later, the transgenic cells suspended in PBS were used to immunize the mice four times at three-day intervals, using 1 x 10⁶ cells for each immunization. Two days after the final immunization, the lymph nodes were extirpated from the mice, a P3U1 myeloma cell line was added at 1/3 the equivalent of the total number of lymphocytes, and cell fusion was carried out using polyethylene glycol (WAKO). The fused cells were cultured for two weeks in HAT medium (GIBCO) to select only the fused cells. Flow cytometry was used to confirm the reactivity of culture supernatant of the obtained fused cells (hybridomas) toward the transgenic cells, and highly reactive hybridomas were subcultured. The hybridomas were cultured in 1 L of 10% FBS-RPMI medium, the culture supernatant was dialyzed against PBS, and then adsorbed onto a Protein A column (Amersham). The adsorbed monoclonal antibodies were eluted with 0.17 M glycine-HCl buffer (pH4.0), and the eluted fractions were combined and dialyzed against PBS. Some of the monoclonal antibodies were biotinylated using EZ-Link Biotinylation Kit (PIERCE) with the aim of confirming their flow cytometry reactivity.

### 6. Flow cytometry and cell sorting

The monoclonal antibodies used for flow cytometry and cell sorting included CD45-PE, CD45-FITC, CD117-PE, CD34-PE, IgG2a-FITC, IgG1-FITC, IgG2a-PE, IgG1-PE, and streptavidin FITC; all were products from Immunotech. The frozen cells were thawed at 37°C, washed with IMDM medium (SIGMA) containing 10% FBS (MBL), and then suspended in 5% FBS-PBS. 50 µg/mL of biotin-labeled PCLP1 antibody and commercially available PE-labeled antibody were simultaneously reacted in ice for one hour. The cells were washed several times in 5% FBS-PBS, and then reacted with streptavidin-FITC for 20 minutes in ice. The cells were washed and then suspended in 5% FBS-PBS to a concentration of 5 x 10⁶ cells/mL. Analysis and cell fractionation were carried out using a Beckman Coulter Epics Altra.

### Results:

### 1. Establishment of a cell line in which human PCLP1 protein is forcibly expressed

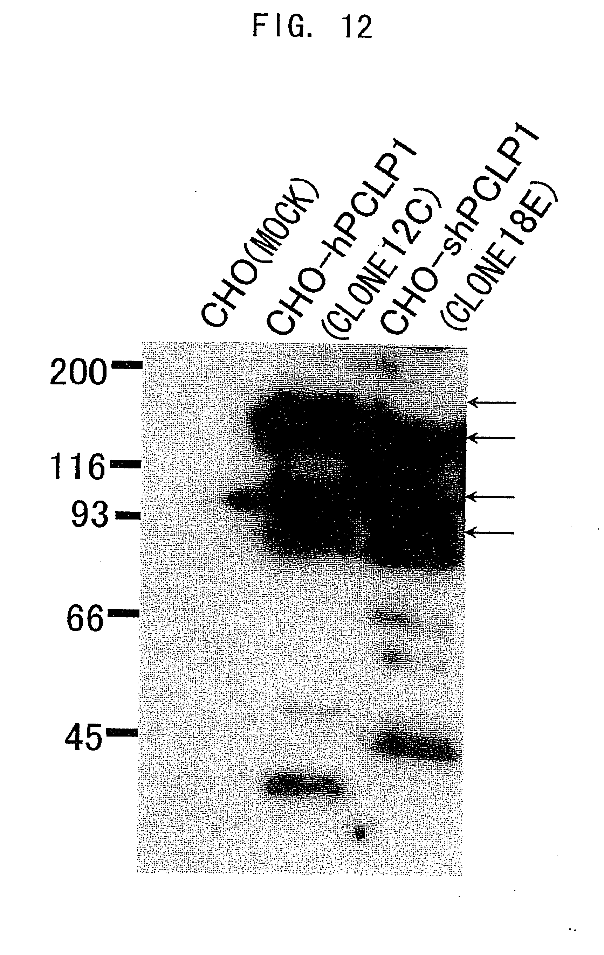

Full-length and extramembrane region PCLP1 genes were forcibly expressed in CHO cells, and cell lines each stably expressing the recombinant proteins were established (Fig. 12). The full length PCLP1-expressing cell line, clone 12C, was used as an immunogen when producing monoclonal antibodies and for confirming reactivity in flow cytometry, and the 18E cell line, which expresses extramembrane PCLP1, was used to purify recombinant proteins from cell cultures. This confirmed that the recombinant protein could be concentrated from the 18E cell culture medium by using a support (Sepharose) to which are bound proteins that recognize sugar chains, such as WGA and ConA (Fig. 13).

### 2. Production of monoclonal antibodies that recognize human PCLP1

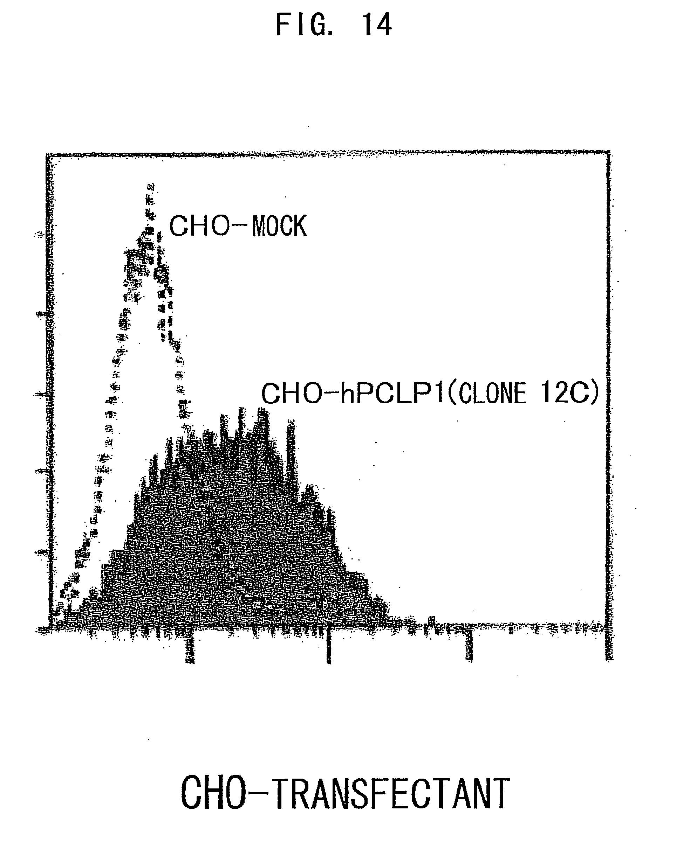

Hybridomas (clone 53D11 and such) that produce anti-human PCLP1 monoclonal antibodies were established by immunizing mice with the gene expression cell line. Anti-human PCLP 1 monoclonal antibodies were purified from the hybridoma culture supernatant to produce biotinylated antibodies and the like. The obtained antibodies were confirmed to have reactivity against the cell line (12C) expressing the full-length human PCLP1 protein (Fig. 14).

### 3. Confirming the reactivity of anti-human PCLP1 monoclonal antibodies

The produced monoclonal antibodies were confirmed to have reactivity against bone marrow (Fig. 15). Cell separation from the bone marrow cells of human individuals clarified that cells can actually be separated using these antibodies (Fig. 15). The cell population that reacted with the anti-human PCLP1 antibodies was confirmed to partly overlap with the known hematopoietic stem cell population (CD34-positive cells), but was largely different.

### Discussion:

Monoclonal antibodies that recognize the human PCLP1 molecule were produced using materials in which recombinant proteins are expressed in animal cells. The percentage of PCLP1 molecule-expressing cells in the human bone marrow is very low, less than 1%, and the distribution of these cells hardly overlaps with the distribution of the hematopoietic stem cell (CD34) population. This finding matches the expression pattern in murine bone marrow.

### [Example 4] Analysis of the relationship between CD34-positive, c-Kit-positive population and PCLP1

### Materials:

C57BL/6 mice
PBS
Collagenase/Dyspase solution (GEBCO BRL)
50 µg/mL gentamicin/15% FBS/DMEM (GIBCO BRL)
2% FBS/PBS, OP9 cell line
Anti-mouse CD16/32 monoclonal antibody (Pharmingen)
Biotinylated anti-mouse PCLP1 monoclonal antibody (MBL)
APC-labeled anti-mouse c-Kit monoclonal antibody (Pharmingen)
FITC-labeled anti-mouse CD34 monoclonal antibody (Pharmingen)
Streptavidin-APC (Molecular Probes)
7-AAD (Pharmingen)
Oncostatin M (OSM), Basic fibroblast growth factor (bFGF)
Stem cell factor (SCF)
Various antibodies against mouse cell surface antigens
MethoCult (Stem Cell Technologies)

### Methods:

### 1. Preparation of newborn bone marrow cells

The mice were euthanized by being left in ice for ten minutes. Their femurs were extirpated under a stereoscopic microscope. The femurs of six to twelve individuals were soaked in 12 mL of Collagenase/Dyspase solution, and then broken into pieces using a pair of tweezers. This was incubated in a CO₂ incubator at 37°C for ten minutes, and the whole, including the bones, was subjected to enzyme treatment. The cells were suspended by thorough pipetting using a 10 mL glass pipette, and then filtered for transfer into a centrifuge tube. An equivalent amount of 50 µg/mL gentamicin/15% FBS/DMEM was added and mixed in, and this was centrifuged at 4°C and 1200 rpm for ten minutes. The supernatant was removed, the residue was resuspended in 50 µg/mL gentamicin/15% FBS/DMEM, and the number of cells was counted using a hemocytometer.

### 2. Antibody reaction

Anti-mouse CD16/32 monoclonal antibody was diluted 100 times with 50 µg/mL gentamicin/15% FBS/DMEM, and 0.1 mL of this solution was added to every 1 x 10⁶ bone marrow cells and mixed. The mixture was left on ice for 15 minutes, and non-specific antibody binding was inhibited by FcR blocking. About 1 x 10⁵ cells were placed into each of four tubes, and the antibodies below were added to the respective tubes and mixed to produce isotype controls and samples for fluorescence correction. Each of the antibodies was added such that they were diluted 100 times.
Tube 1: FITC-labeled rat IgG2a, PE-labeled rat IgG2a, and biotinylated rat IgG2a
Tube 2: FITC-labeled anti-mouse CD45 monoclonal antibody, PE-labeled rat IgG2a, and biotinylated rat IgG2a
Tube 3: FITC-labeled rat IgG2a, PE-labeled anti-mouse CD45 monoclonal antibody, and biotinylated rat IgG2a
Tube 4: FITC-labeled anti-rat IgG2a, PE-labeled rat IgG2a, and biotinylated anti-mouse CD45 monoclonal antibody

Biotinylated anti-mouse PCLP1 monoclonal antibody, APC-labeled anti-mouse c-Kit monoclonal antibody, and FITC-labeled anti-mouse CD34 monoclonal antibody were added to the remaining cells such that they were each diluted 100 times, and this was then mixed to prepare the samples. These were left on ice for 30 minutes, and then the isotype controls, fluorescence correction samples, and samples were each washed with ice-cooled 2% FBS/PBS. The isotype controls, fluorescence correction samples, and samples were each rediluted in streptavidin-APC diluted 50 times with 2% FBS/PBS, and then left in ice for 30 minutes. They were then washed with ice-cooled 2% FBS/PBS. Cells were diluted at 1 x 10⁶ cells per 5 µL 7-AAD, and then left at room temperature for five minutes. The cells were diluted in 2% FBS/PBS or diluted in PBS to a concentration of 2 x 10⁶ to 5 x 10⁶ cells/mL, and then transferred to a cell separator tube.

### 3. Sorting

Using isotype controls and fluorescence correction samples, the sensitivity of each parameter of the cell separator (cell sorter) was adjusted and fluorescence corrections were made. With reference to the fluorescence intensity of the isotype control, the CD34-positive, c-Kit-positive cell population in the sample was gated, and gates were further developed within these gates according to PCLP1 expression levels; two sorting gates were set, one for CD34-positive, c-Kit-positive, and PCLP1-positive cells; and another for CD34-positive, c-Kit-positive, and PCLP1-negative cells. The sorting gates were set such that when the CD34-positive, c-Kit-positive cell fraction is defined as 100%, the PCLP1-negative subfraction will be approximately 58% and the PCLP1-positive subfraction will be approximately 15%. The cells were fractionated in tubes containing 50 µg/mL gentamicin/15% FBS/DMEM. The fractionated cells were reanalyzed to confirm that they were purely fractioned according to the gates that were set. After fractionation the cells were centrifuged and the supernatant removed to reduce the volume of the solution, as necessary. The number of obtained cells was counted using a hemocytometer.

### 4. Co-culturing with OP9 stromal cells

On a 10-cm dish or 6-well plate, OP9 stromal cells were cultured in 50 µg/mL gentamicin/15% FBS/DMEM until approximately 70% to 90% confluent. Immediately before plating the sorted cells onto this dish or plate, the medium was replaced with a medium containing cytokines (10 µg/mL OSM, 1 µg/mL bFGF, and 100 µg/mL SCF). The CD34-positive, c-Kit-positive, and PCLP1-positive fraction, and the CD34-positive, c-Kit-positive, and PCLP1-negative fraction obtained by sorting were each plated into 6-well plates at 3000 cells per well. The cells were cultured in a CO₂ incubator at 37°C under 5% CO₂ partial pressure conditions, and blood cell production in each of the fractions was observed under a microscope for several weeks, starting from the day after plating.

### 5. Analysis of suspended cells produced by OP9 co-culture

After several days of OP9 co-culture, and after producing a sufficient amount of suspended cells, the suspended cells were exclusively collected into centrifuge tubes, taking care to avoid OP9 contamination. The obtained cells were used to analyze the expression of cell surface antigens using flow cytometry, and to measure growth activity of the blood cells using colony assays.

### Results:

### 1. Expression patterns of PCLP1, c-Kit, and CD34 in the bone marrow

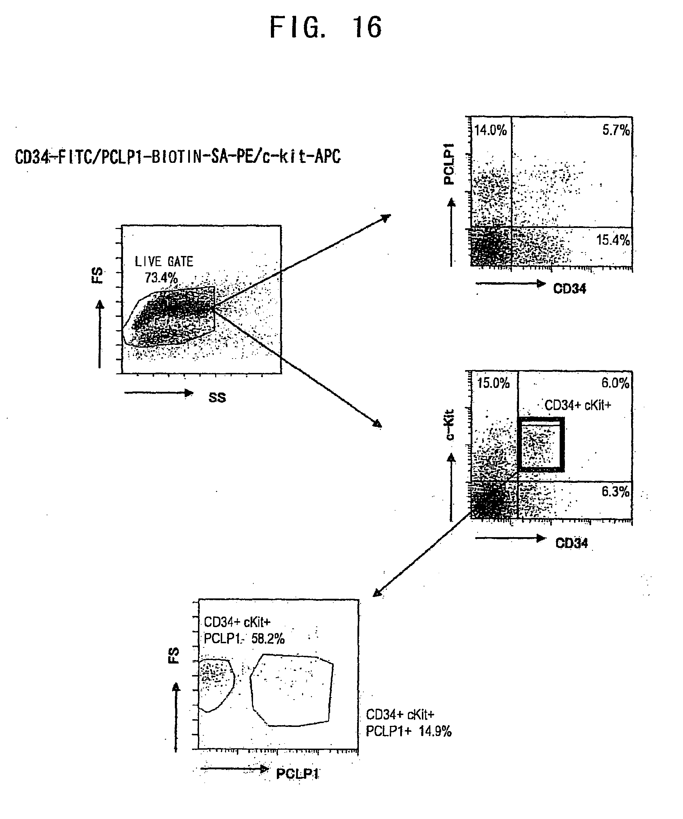

Expression of PCLP 1 in the bone marrow could be categorized into four groups according to expression intensity: strongly positive (approximately 1%), moderately positive (approximately 14%), weakly positive (approximately 8%), and negative (approximately 77%) (Fig. 7). A known hematopoietic stem cell fraction that is CD34-positive and c-Kit-positive was detected as a distinct group constituting about 6% of the cells, and PCLP expression in this fraction was mostly negative; however, a slight distribution was observed in the weakly positive or positive regions (Fig. 16). A similar trend was also found in human bone marrow (Fig. 15).

### 2. Results of co-culturing with OP9 stromal cells

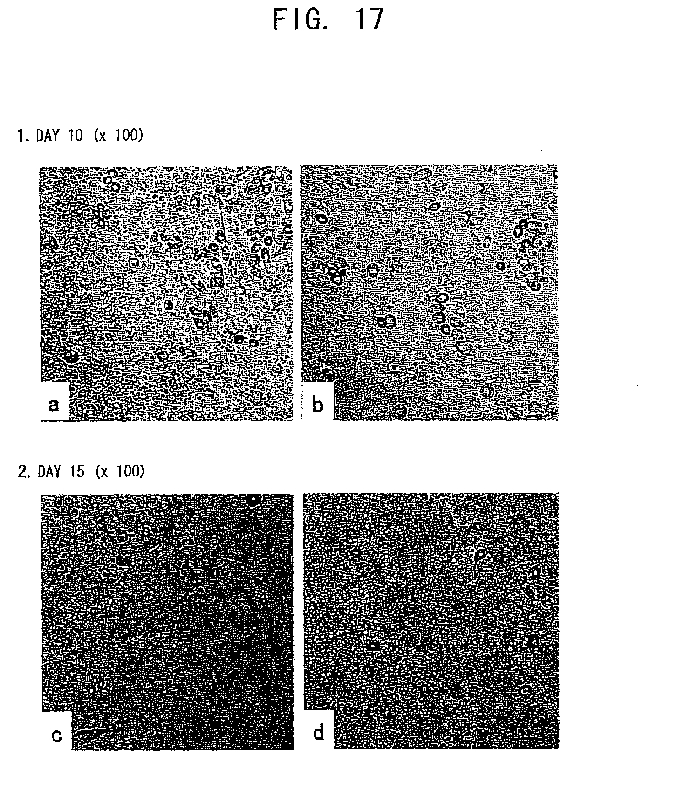

Observation of each fraction under a phase contrast microscope after OP9 co-culture showed that during Days 7 to 14 of culture, the CD34-positive, c-Kit-positive, and PCLP1-positive fraction formed blood cell clusters at a few places in the dish, with growth of suspended blood cell-like cells observed to a degree. In contrast, the CD34-positive, c-Kit-positive, and PCLP1-negative fraction produced such a large amount of suspended blood cell-like cells that the OP9 stromal cells could not be observed (Figs. 17a, b). In this PCLP1-negative fraction, so many blood cells were produced that the culture supernatant was exchanged about twice in the second week of culturing to avoid reducing the biological activity of the culture system due to excessive increase in the number of cells present in the culture solution.

However, by the beginning of the third week of culture, the blood cell growth activity in these two fractions was reversed, and blood cell production gradually ceased in the PCLP1-negative fraction, while blood cell production gradually became more active in the PCLP1-positive fraction (Figs. 17c, d). When suspended cells were collected from both fractions on Day 15 of culture, the number of cobble stone-like cells, which appear black as they crawl under OP9, was obviously much greater in the PCLP1-positive fraction than in the PCLP1-negative fraction.

### 3. Results of analyzing suspended cells produced by OP9 co-culture

When suspended cells of both fractions were each collected on Day 15 of OP9 co-culture, a sufficient amount of cells could not be obtained from the PCLP1-negative fraction, therefore, analysis of cell surface antigen expression by flow cytometry was only carried out for the PCLP1-positive fraction. The results showed that nearly 100% of cells expressed CD45, and that cell surface antigens of hematopoietic stem cells and hematopoietic precursor cells, such as CD34, c-Kit, and CD31, were expressed very frequently (Fig. 18). The PCLP1-positive fraction, in which blood cell growth activation was delayed, maintained its blood cell growth activity for several weeks thereafter.

### 4. Results of colony assays of suspended cells produced by OP9 co-culture

When suspended cells of both fractions were each collected on Day 15 of OP9 co-culture, colony assays were performed, and the results were that CFU-C=753.3 per 10 000 cells regarding the suspended cells derived from the PCLP1-positive fraction, and CFU-C=1583.3 per 10 000 cells regarding the suspended cells derived from the PCLP1-negative fraction (Fig. 19).

### Discussion:

The use of the OP9 co-culture system indicated that bone marrow cells can also grow for a long time *ex vivo* or *in vitro.* The CD34-positive, c-Kit-positive cell fraction are thought to be a fraction with hematopoietic stem cells and hematopoietic precursor cell fractions concentrated to some degree; however, when this fraction was further fractioned into PCLP1-positive and PCLP1-negative subfractions, the time required for blood cell growth to start was significantly different for each of the fractions, and therefore the subfractions are highly likely to be at different differentiation stages of blood cell differentiation.

That is, in hematopoietic stem cell and hematopoietic precursor cell fractions, the low frequency cell populations expressing PCLP1 may be more juvenile. However, the colony forming ability of suspended cells on Day 15 of culture was twice as much or more for cells derived from the PCLP1-negative fraction than for the PCLP1-positive fraction. Since only the PCLP1-positive fraction continued to produce blood cells for several weeks thereafter, it is thought that the above results were because at this point the PCLP1-positive fraction may not have reached the differentiation stage of blood cell differentiation with highest growth activity.

In the AGM region, where hematopoiesis is said to begin during embryonic development, approximately 90% of CD34-positive cells express PCLP1 (WO 01/34797). The present results further confirmed that as the site of hematopoiesis gradually shifts from the AGM to the fetal liver and bone marrow, the proportion of PCLP1-positive cells among the CD34-positive cells dramatically decreases from 50% or so in the fetal liver to a few percent or so in the bone marrow (Fig. 1 and Fig. 16). The distribution in human bone marrow almost matches the results obtained from mice (Fig. 1 and Fig. 15). This showed that the CD34-positive cell population, which had so far been thought to be a population of hematopoietic stem cells, should actually be referred to as a cell population comprising blood cells that have somewhat differentiated towards blood cells, or blood cell precursor cells, and the population of cells that should be referred to as the true hematopoietic stem cells among the CD34-positive cell population can be thought to be the CD34-postive, PCLP1-positive population.

Experimental results supporting this theory include the phenomenon that in a system of co-culture with stromal cells, in terms of the time taken until hematopoietic activity is observed, the CD34-positive, c-Kit-positive, PCLP1-negative cells start to show blood cell growth at a relatively early stage and complete the growth in a short time, whereas, the CD34-positive, c-Kit-positive, PCLP1-positive cell population takes a long time until blood cell growth starts and continues to produce blood cells for a long time (Fig. 17). Further, the blood cells obtainable by co-culturing the CD34-positive, c-Kit-positive, PCLP1-positive fraction with stromal cells are CD34-positive, c-Kit-positive, PCLP1-negative, and thus it is understood that they are the actual stem cells which can form the CD34-positive, c-Kit-positive cell population thought until now to be stem cells (Fig. 18).

### [Example 5] Isolation culture of hematopoietic precursor cells and endothelial precursor cells using spleens from murine individuals

### Materials:

Newborn C57BL/6 mice
PBS
Collagenase/Dyspase solution (GIBCO BRL)
50 µg/mL gentamicin/15% FBS/DMEM (GIBCO BRL)
2% FBS/PBS
OP9 cell line
Anti-mouse CD16/32 monoclonal antibody (Pharmingen)
Biotinylated anti-mouse PCLP1 monoclonal antibody (MBL)
APC-labeled anti-mouse c-Kit monoclonal antibody (Pharmingen)
FITC-labeled anti-mouse CD34 monoclonal antibody (Pharmingen)
Streptavidin-APC (Molecular Probes)
7-AAD (Pharmingen)
Oncostatin M (OSM)
Basic fibroblast growth factor (bFGF)
Stem cell factor (SCF)
Various antibodies against mouse cell surface antigens
MethoCult (Stem Cell Technologies)

### Methods:

### 1. Preparation of spleen cells from individuals

The newborn mice were euthanized by being left in ice for ten minutes. Their spleen was extirpated under a stereoscopic microscope. The spleens were soaked in Collagenase/Dyspase solution at 12 mL of Collagenase/Dyspase solution for a litter of fetuses (six to twelve fetuses), and then cut into small pieces using a pair of surgical scissors. This was incubated in a CO₂ incubator at 37°C for ten minutes, and then subjected to enzyme treatment.

Cells were dispersed by thorough pipetting using a 10-mL glass pipette. These cells were transferred to a centrifuge tube, an equivalent amount of 50 µg/mL gentamicin/15% FBS/DMEM was added and mixed, and this was centrifuged at 4°C and 800 rpm for ten minutes. The supernatant was removed, the residue was resuspended in 50 µg/mL gentamicin/15% FBS/DMEM, and the number of cells was counted using a hemocytometer.

### 2. Antibody reaction

Anti-mouse CD16/32 monoclonal antibody was diluted 100 times with 50 µg/mL gentamicin/15% FBS/DMEM, and 0.1 mL of this solution was added per 1 x 10⁶ spleen cells. This was then mixed, the mixture was left on ice for 15 minutes, and non-specific antibody binding was inhibited by FcR blocking. About 1 x 10⁵ cells were placed into each of four tubes, and the antibodies below were added to the respective tubes and then mixed to produce isotype controls and samples for fluorescence correction. Each of the antibodies was added such that they were diluted 100 times.
Tube 1: FITC-labeled rat IgG2a, PE-labeled rat IgG2a, and biotinylated rat IgG2a
Tube 2: FITC-labeled anti-mouse CD45 monoclonal antibody, PE-labeled rat IgG2a, and biotinylated rat IgG2a
Tube 3: FITC-labeled rat IgG2a, PE-labeled anti-mouse CD45 monoclonal antibody, and biotinylated rat IgG2a
Tube 4: FITC-labeled anti-rat IgG2a, PE-labeled rat IgG2a, and biotinylated anti-mouse CD45 monoclonal antibody

Biotinylated anti-mouse PCLP1 monoclonal antibody, APC-labeled anti-mouse c-Kit monoclonal antibody, and FITC-labeled anti-mouse CD34 monoclonal antibody were added to the remaining cells such that they were each diluted 100 times, and were then mixed to prepare the sample. After adding the antibodies, the respective cells were left on ice for 30 minutes.

The isotype controls, fluorescence correction samples, and samples were each washed with ice-cooled 2% FBS/PBS. The isotype controls, fluorescence correction samples, and samples were each rediluted with streptavidin-APC diluted 50 times with 2% FBS/PBS, and then left in ice for 30 minutes. They were then washed with ice-cooled 2% FBS/PBS. Cells were diluted at 1 x 10⁶ cells per 5 µL of 7-AAD, and then left at room temperature for five minutes. The cells were diluted in 2% FBS/PBS or diluted in PBS to a concentration of 2 x 10⁶ to 5 x 10⁶ cells/mL, and then transferred to a cell separator tube.

### 3. Sorting (Cell separation)

Using isotype controls and fluorescence correction samples, the sensitivity of each parameter of the cell separator was adjusted and fluorescence corrections were made. With reference to the fluorescence intensity of the isotype control, cell population in the sample that was CD34-positive and c-Kit-positive were gated, and gates were further developed within this gate according to PCLP1 expression intensity, with sorting gates set for the following three regions:
CD34-positive, c-Kit-positive, and PCLP1-positive;
CD34-positive, c-Kit-positive, and weakly PCLP1-positive; and
CD34-positive, cKit-positive, and PCLP1-negative.

The sorting gates were set such that when the CD34-positive and c-Kit-positive cell fraction was defined as 100%, the PCLP1-positive subfraction was approximately 16%, the weakly PCLP1-positive subfraction was approximately 13%, and the PCLP1-negative subfraction was approximately 71%. The samples were developed according to the fluorescence intensity of PCLP1 and cell size (FS peak). With reference to the fluorescence intensity of the isotype control, strongly PCLP1-positive, moderately PCLP1-positive, weakly PCLP1-positive, and PCLP1-negative regions were each gated.

The cells were fractionated into tubes containing 50 µg/mL gentamicin/15% FBS/DMEM mixed with 10 µg/mL OSM, 1 µg/mL bFGF, and 100 µg/mL SCF. The fractionated cells were reanalyzed to confirm that they were purely fractioned according to the gates that were set. The number of obtained cells was counted using a hemocytometer.

### 4. Co-culturing with OP9 stromal cells

On a 10-cm dish or 6-well plate, OP9 stromal cells were cultured in 50 µg/mL gentamicin/15% FBS/DMEM until 70% to 90% confluent. Immediately before plating the sorted cells onto this dish or plate, the medium was replaced with that containing cytokines (10 µg/mL OSM, 1 µg/mL bFGF, and 100 µg/mL SCF). The following cells obtained by sorting were plated:
CD34-positive, c-Kit-positive, and PCLP1-positive fraction (2600 cells/well);
CD34-positive, c-Kit-positive, and weakly PCLP1-positive fraction (2600 cells/well);
CD34-positive, cKit-positive, and PCLP1-negative fraction (2600 cells/well); and
Strongly PCLP1-positive cells (2000 cells/well).

The cells were cultured in a CO₂ incubator at 37°C under 5% CO₂ partial pressure conditions. Blood cell production in each of the fractions was observed under a microscope for several weeks, starting from the day after plating.

### Results:

### 1. Expression pattern of PCLP1, c-Kit, and CD34 in the spleen

The expression of PCLP1 in the spleen could be categorized into four groups according to expression intensity:
Strongly positive (PCLP1++; approximately 1%);
Moderately positive (PCLP1+; approximately 30%);
Weakly positive (PCLP1 low; approximately 18%); and
Negative (PCLP1-; approximately 51%).

This pattern of PCLP1 expression was similar to the pattern of PCLP1 expression in the E14.5 fetal liver. On the other hand, the fraction of CD34-positive, c-Kit-positive cells was detected to be a distinct group constituting 5% or so of cells, and PCLP 1 expression in this fraction was mostly negative; however, a slight distribution was observed in the weakly positive or positive regions.

### 2. Results of co-culturing with OP9 stromal cells

Observation of OP9 co-cultures of each fraction under a phase-contrast microscope showed that the CD34-positive, c-Kit-positive, and PCLP1-negative fraction actively produced blood cell-like cells for the first two to three weeks of culture. Blood cell growth in the weakly PCLP1-positive fraction was somewhat weaker than for the strongly positive fraction, and the growth was even weaker in the PCLP 1-positive fraction (Figs. 20c-e). However, after the first month of culture, this activity of blood cell growth was reversed, and blood cell production gradually ceased in the PCLP1-negative fraction, and gradually became more active in the PCLP1-positive fraction.

In the strongly PCLP1-positive fraction, formation of many endothelial cell-like colonies, which were morphologically similar to the endothelial cell-like colonies formed from an OP9 co-culture of strongly PCLP1-positive fetal liver cells, were observed on Day 10 of culture (Figs. 20a, b).

### Discussion:

The results showed that strongly PCLP1-positive cells also exist in the spleen, although in low frequency, and that endothelial-like colonies are produced, which are morphologically similar to those produced by OP9 co-culture of strongly PCLP1-positive cells of the fetal liver. Further, as for fetal liver, blood cell growth for PCLP1-positive cells was activated later than for PCLP1-negative cells.

These results showed that during the developmental process of a fetus, as the site of hematopoiesis shifts from the AGM region, where adult-type hematopoiesis begins, to the liver and spleen, a strongly PCLP1-positive cell fraction and a moderately PCLP1-positive cell fraction are consistently present in each of the hematopoietic organs, and throughout the transition, the strongly PCLP1-positive cell fraction consistently includes a high frequency of endothelial precursor cells, and the moderately positive cells include hematopoietic stem cell-like juvenile cells that continuously produce blood cells for a long time.

### [Example 6] Method of recovering vascular endothelial precursor cells from the bone marrow Materials:

PBS
70% Ethanol
50 µg/mL gentamicin/15% FBS/DMEM (GIBCO BRL)
ACK Buffer: produced by sterilizing the following stock buffers and then mixing them at an A' to B ratio of 9:1.
   Stock buffer A': 155 mM NH₄Cl, 10 mM KHCO₃, 1 mM EDTA-2Na
   Stock buffer B: 0.17 M Tris-HCl (pH 7.65)
5% FBS/PBS
FcR blocker (Pharmingen)
Biotinylated anti-mouse PCLP1 monoclonal antibody (MBL)
Streptavidin magnet beads (Miltenyi Biotec)
SCF
bFGF
mOSM
OP9
Apparatuses
Dissection table, scissors, tweezers, Kimwipes, Falcon tubes, 1-mL syringes (Terumo), 18G injection needles (Terumo), cell strainer (Falcon)
Auto MACS (Miltenyi Biotec).
CO₂ incubator (SANYO)

### Method:

### 1. Collection of bone marrow

Fifty C57BL/6j mice aged three months or more were anesthetized and then subjected to cervical dislocation. The mice were laid on their backs on a dissection table and sprayed thoroughly with 70% ethanol. Using a pair of scissors, an incision was made in the skin of the leg, and excessive fat and muscle was cut out. The joint was dislocated by holding the base of the leg with a pair of scissors, and the femur was extirpated and rubbed thoroughly using Kimwipes to remove unnecessary flesh. Both ends of the femur were cut using a pair of scissors, and a needle was attached to a syringe to draw in a suitable amount of medium. Using a pair of tweezers, the femur was held above a 50-mL Falcon tube containing medium. The tip of the needle was placed into the bone, and one push of the piston was used to push out the femur bone marrow.

### 2. Sample preparation

The tube in which the bone marrow was collected was centrifuged at 1200 rpm for five minutes and the supernatant was discarded, 20 mL of ACK buffer was added and mixed by pipetting, and the mixture was left on ice for ten minutes. An equivalent amount of medium was added and mixed by pipetting. This was transferred to a 50-mL Falcon tube set with a cell strainer to remove excess tissues and unwanted particles, and centrifuged at 1200 rpm for five minutes. The supernatant was discarded, medium was added and mixed by pipetting, and this was centrifuged again at 1200 rpm for five minutes. The supernatant was discarded, 10.5 mL of medium was added, and the cells were suspended. The cell suspension was passed through a cell strainer. The number of cells was counted, and some were transferred to a separate tube and then stored. Ten µL of FcR blocker was added for every 1 x 10⁷ cells/mL, and this was allowed to react on ice for 15 minutes. Anti-mouse PCLP1 antibody was added to a final concentration of 20 µg/mL, and was allowed to react on ice for 30 minutes. Medium was then added to fill the tube to 15 mL, and this was centrifuged at 1200 rpm for five minutes. The supernatant was discarded, medium was again added to fill the tube to 15 mL, and this was centrifuged at 1200 rpm for five minutes. The supernatant was discarded, and streptavidin-magnet beads were added at 4 beads/cell. This was left on ice for ten minutes, then medium was added. This was centrifuged at 1200 rpm for five minutes, the supernatant was discarded, and medium was added again. This was centrifuged at 1200 rpm for five minutes, the supernatant was discarded, the cells were suspended in PBS +5% FBS, and the cell suspension was passed through a cell strainer.

### 3. Cell separation by AutoMACS

Cells were separated on AutoMACS by selecting the POSSELD2 program, and PCLP1-positive cells and PCLP1-negative cells were collected in separate Falcon tubes.

### 4. Co-culturing (Operations 1 and 2 were performed by the day before co-culture)

OP9 cells were seeded at 1 x 10⁴ cells per well on a 6-well plate, and cultured overnight at 37°C. Cytokines (10 ng/mL of OSM, 100 ng/mL of SCF, and 1 ng/mL of bFGF) were added to the medium. PCLP1-positive cells, PCLP1-negative cells, and unseparated cells were each plated at 1 x 10⁴ cells per well, and then cultured at 37°C.

### Results:

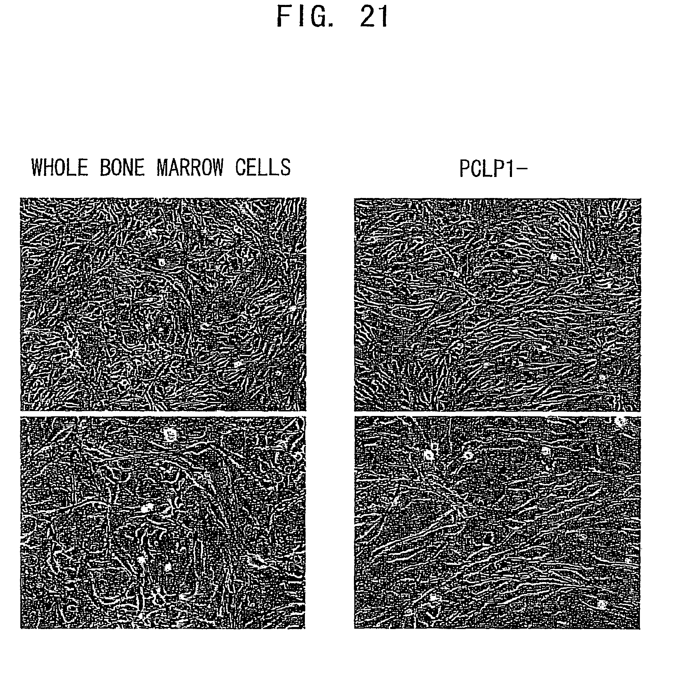

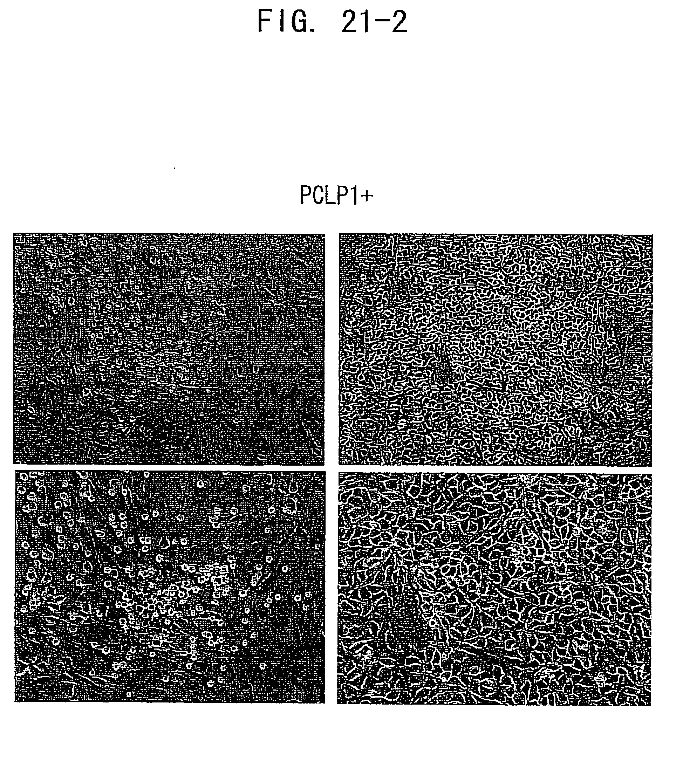

One hundred femurs were extirpated from fifty C57BL/6J mice, and bone marrow cells were separated. The number of obtained bone marrow cells was 1.1 x 10⁹ whole bone marrow cells. When PCLP1-positive cells were separated from the obtained bone marrow using AutoMACS, 2.6 x 10⁶ PCLP1-positive cells were obtained. Whole bone marrow cells, PCLP1-positive cells, and PCLP1-negative cells were each co-cultured with OP9 stromal cells, but on Day 8 of culture only those wells seeded with PCLP1-positive cells were confirmed to form cobble-stones of hematopoietic stem cell growth and have endothelial precursor cell-like colonies (Fig. 21-2). Cobble-stone formation and endothelial precursor cell-like colonies did not occur in the whole bone marrow cell cultures (Fig. 21, left) or PCLP1-negative cell cultures (Fig. 21, right).

The above-mentioned results showed that endothelial precursor cells exist in the bone marrow of individuals, although in low frequency, and that a cell population that differentiates into endothelial precursor cells can be separated using anti-PCLP1 monoclonal antibodies.

### Industrial Applicability

The hematopoietic stem cells obtainable by the present invention are useful for treating various blood diseases. Specific examples include leukemia and immunodeficiency. In such diseases the hematopoietic system of a patient is reconstructed by autotransplantation or allotransplantation of the hematopoietic stem cells obtained by the present invention to the patient, enabling radical cure of the above-mentioned diseases. The present invention enables amplification of hematopoietic stem cells *in vitro,* and since introduction of genes is highly possible during this process, the present invention thus provides very useful methods for stem cell transplantation and gene therapy for blood diseases.

On the other hand, vascular endothelial precursor cells obtainable by the present invention are useful for treating vascular diseases. Specific examples include arteriosclerosis obliterans and myocardial infarction. Such diseases may be radically cured by regenerating new blood vessels in place of obstructed arteries, and by regenerating damaged vascular endothelial cells to reestablish sufficient blood flow. Such attempts have been made in the past using bone marrow cells, however, since bone marrow cells include only a small number of vascular endothelial precursor cells and also include cells that may differentiate into bone, muscle, adipocytes, and such, risks have been pointed out regarding methods for direct transplantation of bone marrow cells. Since the present invention comprises the steps of isolating vascular endothelial precursor cells, and amplifying them by culturing the cells *in vitro,* it may enable selective transplantation of vascular endothelial cells. Suppression of angiogenesis by the *in vitro* culture system of vascular endothelial precursor cells of the present invention may also be a method useful for developing anticancer agents which have the effect of protecting against the malignant transformation of cancers.

## Claims

1. A method for producing a hematopoietic stem cell or a vascular endothelial precursor cell, wherein the method comprises the steps of:
(1) separating a PCLP1-positive cell from a hematopoietic tissue of an individual;
(2) inducing a hematopoietic stem cell or a vascular endothelial precursor cell by culturing the PCLP1-positive cell; and
(3) collecting the hematopoietic stem cell or vascular endothelial precursor cell from the culture of (2).

2. The method of claim 1, wherein the PCLP1-positive cell is a c-Kit-positive cell, and the method comprises the step of collecting the hematopoietic stem cell.

3. The method of claim 1, wherein the PCLP1-positive cell is an erythroblast cell surface antigen-negative cell, and the method comprises the step of collecting the vascular endothelial precursor cell.

4. The method of claim 3, wherein the PCLP1-positive cell is an erythroblast cell surface antigen-negative and CD45-negative cell.

5. The method of claim 1, wherein the hematopoietic tissue is bone marrow.

6. The method of claim 5, which comprises the step of collecting a vascular endothelial precursor cell.

7. The method of claim 5, which comprises the step of collecting a hematopoietic stem cell.

8. The method of claim 5, wherein the PCLP1-positive cell is a CD34-positive cell.

9. The method of claim 1, wherein the hematopoietic tissue is spleen tissue.

10. The method of claim 9, which comprises the step of collecting a vascular endothelial precursor cell.

11. The method of claim 9, which comprises the step of collecting a hematopoietic stem cell.

12. The method of claim 1, wherein step (2) is the step of co-culturing a PCLP1-positive cell with a stromal cell.

13. The method of claim 12, wherein a PCLP1-positive cell and a stromal cell are co-cultured in the presence of oncostatin M (OSM), basic fibroblast growth factor (bFGF), and stem cell factor (SCF).

14. The method of claim 1, wherein step (2) is the step of culturing a PCLP1-positive cell in the presence of a humoral factor present in the culture of a stromal cell.

15. A hematopoietic stem cell or vascular endothelial precursor cell produced by the method of claim 1.

16. A kit for producing a hematopoietic stem cell or a vascular endothelial precursor cell, wherein the kit comprises the following elements:
(a) a reagent for detecting the level of PCLP1 expression; and
(b) a medium for culturing a PCLP1-positive cell.

17. The kit of claim 16, which additionally comprises (c) a stromal cell.

18. The kit of claim 16, which additionally comprises (d) a reagent for detecting the level of expression of at least one cell surface antigen selected from the group consisting of an erythroblast cell surface antigen, CD45, and CD34.

19. A method for treating a disease caused by a hematopoietic cell deficiency, wherein the method comprises the step of administering a hematopoietic stem cell obtained by the method of claim 1.

20. A method for supplementing a blood cell, which comprises the step of administering a hematopoietic stem cell obtained by the method of claim 1.

21. A method for treating a vascular disease, which comprises the step of administering a vascular endothelial precursor cell obtained by the method of claim 1.

22. A method for detecting a regulatory effect of a test substance on angiogenic activity, wherein the method comprises the steps of:
(1) culturing a vascular endothelial precursor cell obtained by the method of claim 1 with a test substance;
(2) observing the level of growth of the vascular endothelial precursor cell; and
(3) detecting the regulatory effect of the test substance on angiogenic activity when the level of growth is found to differ from that of a control.

23. The method of claim 22, wherein an inhibitory effect on angiogenesis is detected when the level of growth is decreased.

24. The method of claim 22, wherein an accelerating effect on angiogenesis is detected when the level of growth is increased.

25. A method of screening for a substance with a regulatory effect on angiogenic activity, wherein the method comprises the steps of:
(1) detecting the regulatory effect of a test substance on angiogenic activity as per the method of claim 22; and
(2) selecting a test substance that has a regulatory effect on angiogenic activity.

26. An inhibitor or accelerator of angiogenesis, which comprises a substance selected by the method of claim 25 as an active ingredient.

27. An anticancer agent against a cancer cell caused by angiogenesis, wherein the agent comprises, as an active ingredient, a substance with an inhibitory effect on angiogenic activity, where the substance has been selected by the method of claim 25.

28. A kit for detecting a regulatory effect on angiogenic activity, wherein the kit comprises the following elements:
a) a vascular endothelial precursor cell obtained by the method of claim 1; and
b) a medium for culturing the cell of a).
